(19) Europäisches Patentamt
European Patent Office
Office européen des brevets

(11) EP 2 056 780 B1

(12) # EUROPÄISCHE PATENTSCHRIFT

(45) Veröffentlichungstag und Bekanntmachung des Hinweises auf die Patenterteilung:
**14.08.2013 Patentblatt 2013/33**

(21) Anmeldenummer: **07821176.0**

(22) Anmeldetag: **11.10.2007**

(51) Int Cl.:
***A61K 8/25*** *(2006.01)* ***A61K 8/26*** *(2006.01)*
***A61K 8/46*** *(2006.01)* ***A61K 8/49*** *(2006.01)*
***A61K 8/81*** *(2006.01)* ***A61Q 5/08*** *(2006.01)*

(86) Internationale Anmeldenummer:
**PCT/EP2007/060809**

(87) Internationale Veröffentlichungsnummer:
**WO 2008/055756 (15.05.2008 Gazette 2008/20)**

(54) **REDUKTIVER FARBABZUG**

REDUCTIVE COLOUR REMOVAL

DÉCOLORATION PAR RÉDUCTION

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **10.11.2006 DE 102006053402**

(43) Veröffentlichungstag der Anmeldung:
**13.05.2009 Patentblatt 2009/20**

(73) Patentinhaber: **Henkel AG & Co. KGaA**
**40589 Düsseldorf (DE)**

(72) Erfinder:
- **HÖFFKES, Horst**
**40595 Düsseldorf (DE)**
- **GROSS, Wibke**
**40549 Düsseldorf (DE)**
- **KNÜBEL, Georg**
**40219 Düsseldorf (DE)**
- **MÜLLER, Burkhard**
**21075 Hamburg (DE)**
- **MUCHA, Thomas**
**40699 Erkrath (DE)**

(56) Entgegenhaltungen:
**WO-A-02/30369**
**WO-A-99/18067**
**DE-A1- 1 617 829**
**US-A- 3 892 845**
**US-A1- 2005 169 862**

EP 2 056 780 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft Zusammensetzungen zum reduktiven Farbabzug, welche in einem kosmetischen Träger neben mindestens einem Reduktionsmittel mindestens ein Adsorptionsmittel enthalten. Diese Farbabzugsmittel eignen sich für die kosmetische Entfärbung gefärbter Substrate wie Haut oder keratinhaltige Fasern, beispielsweise menschliche Haare. Gleichfalls ist ein kosmetisches Verfahren zur Entfärbung gefärbter Substrate, sowie die Verwendung besagter Mittel zum Farbabzug Gegenstand der Erfindung.

Schon immer übten gefärbte oder bedruckte Materialien auf den Menschen einen besonderen Reiz aus. Diese Faszination besteht noch heute. Entweder bietet eine Färbung eine modische Komponente, beispielsweise in Form gefärbter Textilien oder gefärbter Haare. Oder die Färbung, beispielsweise in Form eines Drucks, dient der Informationsübermittlung oder der Kunst. Die Färberei fördert kulturelle und soziale Identität und ist darüber hinaus ein Handwerk, welches sich über die Jahre zu einem lukrativen und innovativen Industriezweig gewandelt hat.

Beim Färben wird der Farbstoff auf das Substrat durch Adsorption an die Oberfläche, durch Eindiffundieren, durch Bildung auf und/oder in dem Substrat bzw. durch chemische Bindung übertragen. Zuerst wurden natürliche Farbstoffe, wie z.B. Purpur oder Carmin, verwendet. Durch den rasanten Fortschritt der Wissenschaften wurden über die Jahre für jede Anwendung maßgeschneiderte, synthetische Farbstoffe zugänglich. Für das Färben, beispielsweise von Papier, Textilien oder keratinhaltigen Fasern, kommen im allgemeinen entweder direktziehende Farbstoffe oder Oxidationsfarbstoffe in Frage. Oxidationsfarbstoffe entstehen durch oxidative Kupplung einer oder mehrerer Entwicklerkomponenten untereinander oder mit einer oder mehreren Kupplerkomponenten. Kuppler- und Entwicklerkomponenten werden auch als Oxidationsfarbstoffvorprodukte bezeichnet. Die oxidative Kupplung findet bevorzugt während der Färbevorgangs statt, damit die Farbstoffvorprodukte in das Substrat hinein diffundieren können und der Farbstoff sich in dem Substrat bildet. Durch die Größe des entstandenen Farbstoffmoleküls wird ein Auswaschen aus dem Substrat erschwert.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 4,5-Diamino-1-(2-hydroxyethyl)pyrazol.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere $\alpha$-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Unter direktziehenden Farbstoffen werden im allgemeinen Farbstoffe verstanden, die bereits vor Beginn des Färbens vorgebildet sind und auf das Substrat aufziehen. Wichtige Vertreter dieser Farbstoffklasse sind beispielsweise Triphenylmethanfarbstoffe, Azofarbstoffe, Anthrachinonfarbstoffe oder Nitrobenzolfarbstoffe, welche jeweils kationische oder anionische Gruppen tragen können.

Ein wichtiges technisches Gebiet stellt neben dem Färben das Abziehen von Farbstoffen dar. Darunter versteht man im Allgemeinen das Entfernen von Färbungen oder Bedruckungen durch Auswaschen, chemische Veränderung oder Zerstörung des Farbstoffes. Die oxidative oder die reduktive Entfärbung gefärbter Materialien findet insbesondere bei der Entfärbung von Textilien oder Haaren Anwendung.

Eine oxidative Entfärbung führt meist zu guten Ergebnissen. Jedoch kann durch die starke Oxidationswirkung des zur Entfärbung benutzten Oxidationsmittels die Struktur des Substrats chemisch verändert werden. Dies geht mit einer unerwünschten physikalischen Änderung des Substrats einher. Haare können beispielsweise spröde werden oder insbesondere bei mehrmaliger Entfärbung gar brechen. Der visuelle Eindruck, die Haptik aber auch die Haltbarkeit des Substrats werden dadurch negativ beeinflusst.

Weniger Einfluss auf die Substratstruktur, insbesondere auf die Struktur keratinhaltiger Fasern, nehmen reduktive Entfärbemittel. Die reduktiven Entfärbemittel entfärben kaum die Naturhaarfarbe, sondern wirken lediglich auf die durch Färbung aufgetragenen Farbstoffe reduktiv ein. Es tritt somit kaum eine Aufhellung des Haars auf. Allerdings bedürfen die reduktiven Entfärbemittel einer Verbesserung der Entfärbeleistung.

Überwiegend nutzt man reduktiv wirkende Schwefelverbindungen zur Entfärbung. Bekanntermaßen eignen sich Dithionite oder Derivate der 1-Hydroxymethylsulfinsäure bzw. der 1-Aminomethylsulfinsäure als Reduktionsmittel.

Aus der Druckschrift US-A-3 892 845 sind dem Fachmann reduktive Farbabzugsmittel bekannt, mit deren Hilfe

sich gefärbte keratinhaltige Fasern entfärben lassen. Als Reduktionsmittel ist 1-Hydroxymethylsulfinsäure oder ein Salz davon in den Entfärbemitteln enthalten. Gemäß DE-OS-1 617 829 lässt sich durch Zugabe von 1-Hydroxymethylsulfinsäure zu oxidativen Entfärbemitteln, enthaltend Wasserstoffperoxid und Persulfate, deren Bleichwirkung verstärken.

Laut Offenbarung der EP-A-1 079 018 eignen sich Aminoalkansulfinate der Formel $R1_{3-z}N\ (CR^2R^3\text{-}SO_2\text{-}M)_z$ ($R^2$, $R^3$ = Wasserstoff oder ($C_1$ bis $C_4$)-Alkyl) zur teilweisen Entfärbung von mit Küpenfarbstoffen oder Schwefelfarbstoffen gefärbten oder bedruckten Textilien.

Gemäß WO-A1-99/18067 eignen sich 1-Hydroxyalkylsulfinsäuren bzw. 1-Aminoalkylsulfinsäuren, welche eine Carboxygruppe, eine Sulfonsäuregruppe, eine Acylgruppe, eine Aminocarbonylgruppe oder eine Alkoxycarbonylgruppe tragen, zur Entfärbung gefärbter Substrate. Besagte Derivate besitzen eine vergleichbare oder bessere Entfärbekraft wie die 1-Hydroxymethylsulfinsäure (*vide supra*). In der WO-A1-02/30369 wird die Entdeckung beschrieben, dass sich mit den Sulfinsäure-Derivaten der WO-A1-99/18067 auch keratinhaltige Fasern, wie beispielsweise Haare, entfärben lassen. Weitere zur Entfärbung keratinhaltiger Fasern geeignete Sulfinsäurederivate finden sich in den Druckschriften WO-A1-03/026597 und WO-A1-03/41668.

Substrate, die mit den Sulfinsäurederivaten des Standes der Technik reduktiv entfärbt wurden, erfahren einige Zeit nach dem Farbabzug eine unerwünschte Nachdunkelung.

Aufgabe der vorliegenden Erfindung ist es, in der Entfärbekraft verbesserte reduktive Entfärbemittel bereitzustellen, die Haut und/oder keratinhaltige Fasern, insbesondere menschliches Haar, dauerhaft ohne Nachdunkelung entfärben. Die Substratstruktur soll dabei geschont werden. Ferner sollten die in den Entfärbemitteln eingesetzten Reduktionsmittel für eine kosmetische Verwendung physiologisch verträglich und toxikologisch unbedenklich sein.

Überraschenderweise wird die Aufgabe durch Mittel gelöst, die eine Kombination aus mindestens einem Reduktionsmittel und mindestens einem Adsorptionsmittel enthalten. Die kosmetische Entfärbung von Haut oder keratinhaltigen Fasern, insbesondere menschlichem Haar, gelingt sehr gut ohne dass sich nach dem Entfärbevorgang eine Nachdunkelung in dem hohen Maße einstellt, wie sie durch die Verwendung der Reduktionsmittel allein hervorgerufen wird. Die erfindungsgemäßen Mittel eignen sich besonders zur verbesserten, faserschonenden Entfärbung keratinhaltiger Fasern.

Unter keratinhaltigen Fasern sind beispielsweise Wolle, Pelze, Federn und insbesondere menschliche Haare zu verstehen.

Ein erster Gegenstand der Erfindung sind daher Mittel, enthaltend in einem kosmetischen Träger mindestens ein Reduktionsmittel und mindestens ein Adsorptionsmittel, dadurch gekennzeichnet, dass das Adsorptionsmittel aus Homo - und Copolymeren des Vinylpyrrolidons ausgewahlt wird.

Ein erfindungsgemäß einsetzbares Reduktionsmittel besitzt ein derartiges elektrochemisches Potenzial, dass es ein synthetisches Farbstoffmolekül, insbesondere einen synthetischen Oxidationsfarbstoff, reduzieren und dadurch entfärben kann.

Die Reduktionsmittel sind in den erfindungsgemäßen Mitteln bevorzugt von 2 bis 20 Gew.-%, besonders bevorzugt von 5 bis 10 Gew.-%, jeweils bezogen auf das Gewicht des Mittels, enthalten.

Erfindungsgemäß bevorzugte Reduktionsmittel enthalten mindestens ein Schwefelatom. Besonders bevorzugte Reduktionsmittel im Sinne der Erfindung sind Thionit-Radikal-Abspalter. Diese sind anorganische oder organische Substanzen, die das Radikal-Anion $SO_2^-$ abspalten können.

Erfindungsgemäß bevorzugte Thionit-Radikal-Abspalter werden ausgewählt aus mindestens einem Vertreter aus der Gruppe, die gebildet wird aus Dithionit-Dianion $S_2O_4^{2-}$ und organischen Sulfinsäuren bzw. deren physiologisch verträglichen Salzen.

Als ein bevorzugter anorganischer Thionit-Radikal-Abspalter eignet sich erfindungsgemäß bevorzugt das Dithionit-Dianion $S_2O_4^{2-}$. Letzteres kann in Form eines Salzes, insbesondere eines Metallsalzes wie eines Alkalimetallsalzes und/oder eines Erdalkalimetallsalzes, insbesondere eines Alkalimetallsalzes, als Reduktionsmittel in den erfindungsgemäßen Mitteln enthalten sein.

Als bevorzugte organische Thionit-Radikal-Abspalter lässt sich mindestens eine organische Sulfinsäure bzw. deren physiologisch verträgliches Salz (insbesondere mindestens eine alpha-Hydroxy-sulfinsäure bzw. deren physiologisch verträgliches Salze und/oder mindestens eine alpha-Amino-sulfinsäure bzw. deren physiologisch verträgliches Salz) in den erfindungsgemäßen Mitteln einsetzen.

Besonders bevorzugte erfindungsgemäße Mittel enthalten als organische Sulfinsäure mindestens ein Sulfinsäure-Derivat der Formel (I)

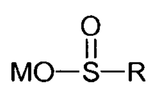

(I)

worin

M steht für ein Wasserstoffatom oder ein Äquivalent eines ein- oder mehrwertigen Kations,

R sich ableitet von einem Peptid oder für einen Rest gemäß einer der Formeln (II) bis (VI) steht,

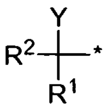 (II)

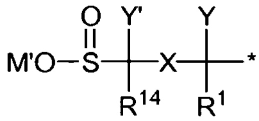 (III)

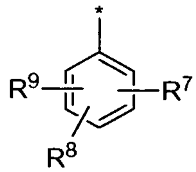

(IV)

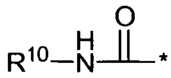 (V)

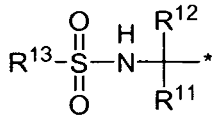 (VI)

worin bedeuten

Y und Y' unabhängig voneinander eine Hydroxygruppe, eine $-NH_2$ Gruppe oder eine Gruppe $-NR^3R^4$, wobei $R^3$ und $R^4$ unabhängig voneinander für eine ($C_1$ bis $C_6$)-Alkylgruppe, eine ($C_2$ bis $C_6$)-Alkenylgruppe, eine ($C_1$ bis $C_6$)-Hydroxyalkylgruppe, eine ($C_2$ bis $C_6$)-Polyhydroxyalkylgruppe, eine Arylgruppe oder eine Aryl-($C_1$ bis $C_6$)-alkylgruppe stehen,

M' unabhängig von M die unter M aufgeführten Merkmale,

X eine direkte Bindung oder einen organischen Rest mit zwei freien Valenzen,

$R^1$ und $R^{14}$ unabhängig voneinander ein Wasserstoffatom, eine ($C_1$ bis $C_6$)-Alkylgruppe oder eine Carboxyalkylgruppe $-(CH_2)_m-COOM^{II}$, in der M" für ein Wasserstoffatom oder für ein Äquivalent eines ein- oder mehrwertigen Kations steht und m die Zahl 0, 1 oder 2 bedeutet,

$R^2$ ein Wasserstoffatom, eine ($C_1$ bis $C_6$)-Alkylgruppe, einen Carboxyalkylrest $-(CH_2)_n-COOM^{III}$ mit $M^{III}$ = Wasserstoffatom oder ein Äquivalent eines ein- oder mehrwertigen Kations und n eine ganze Zahl 0, 1, 2, 3, 4, 5 oder 6, einen ($C_1$ bis $C_6$)-Alkyloxycarbonylrest, eine Sulfonsäuregruppe, eine Carbamoylgruppe, eine N,N-Di[($C_1$ bis $C_6$)-alkyl]carbamoylgruppe, eine N,N,N-Tri[($C_1$ bis $C_6$)-alkyl]ammonium-($C_1$ bis $C_6$)-alkylgruppe eine Carboxy-($C_2$ bis $C_6$)-alkenylgruppe, eine Cyano-($C_1$ bis $C_6$)-alkylgruppe oder eine ($C_1$ bis $C_6$)-Alkoxycarbonyl-($C_1$ bis $C_6$)-alkylgruppe, einen aliphatischen oder aromatischen Heterozyklus, der substituiert sein kann, oder

einen Rest gemäß Formel (IV) oder

$R^2$ zusammen mit $R^1$ und dem Restmolekül einen aliphatischen 5-, 6-, oder 7-gliedrigen Ring bildet, welcher mindestens ein kationisches, quaterniertes Stickstoffatom als Heteroatom enthält, wobei die kationische Ladung gegebenenfalls durch ein Äquivalent eines ein- oder mehrwertigen Anions kompensiert wird,

$R^7$ eine Carboxygruppe, eine Sulfonsäuregruppe, eine ($C_1$ bis $C_6$)-Alkoxycarbonylgruppe, eine Sulfonamidgruppe, eine Cyanogruppe, eine Nitrogruppe, eine Carboxy-($C_1$ bis $C_6$)-alkylgruppe, eine Carboxy-($C_1$ bis $C_6$)-alkoxygruppe oder eine Gruppe $-N^+R^IR^{II}R^{III}$,

mit $R^I$, $R^{II}$ und $R^{III}$ stehen unabhängig voneinander für eine ($C_1$ bis $C_6$)-Alkylgruppe, eine ($C_2$ bis $C_6$)-Alkenylgruppe, eine Aryl($C_1$ bis $C_6$)-alkylgruppe oder eine ($C_1$ bis $C_6$)-Hydroxyalkylgruppe,

$R^8$ und $R^9$ unabhängig voneinander ein Wasserstoffatom, eine ($C_1$ bis $C_6$)-Alkylgruppe, eine ($C_2$ bis $C_6$)-Alkenylgruppe, eine ($C_1$ bis $C_6$)-Hydroxyalkylgruppe, eine ($C_2$ bis $C_6$)-Polyhydroxyalkylgruppe, eine ($C_1$ bis $C_6$)-Alkoxygruppe, eine Hydroxygruppe, eine Aminogruppe, eine Carboxygruppe, eine Nitrogruppe, eine Cyanogruppe oder ein Halogenatom,

$R^{10}$ eine ($C_1$ bis $C_6$)-Alkylgruppe, eine gegebenenfalls substituierte Arylgruppe, eine gegebenenfalls substituierte Heteroarylgruppe, eine Carboxy-($C_1$ bis $C_6$)-alkylgruppe, eine Carboxy-($C_2$ bis $C_6$)-alkenylgruppe, eine ($C_1$ bis $C_6$)-Alkoxycarbonylgruppe oder eine ($C_1$ bis $C_6$)-Alkoxycarbonyl-($C_1$ bis $C_6$)-alkylgruppe,

$R^{11}$, $R^{12}$ und $R^{13}$ unabhängig voneinander ein Wasserstoffatom, eine ($C_1$ bis $C_6$)-Alkylgruppe, eine ($C_2$ bis $C_6$)-Alkenylgruppe, eine Perfluor-($C_1$ bis $C_6$)-alkylgruppe, eine ($C_3$ bis $C_6$)-Cycloalkylgruppe, eine gegebenenfalls substituierte Arylgruppe, eine gegebenenfalls substituierte Heteroarylgruppe, eine ($C_1$ bis $C_6$)-Hydroxyalkylgruppe, eine ($C_2$ bis $C_6$)-Polyhydroxyalkylgruppe, eine Aryl-($C_1$ bis $C_6$)-alkylgruppe, eine Carboxy-($C_1$ bis $C_6$)-alkylgruppe, eine Carboxy-($C_2$ bis $C_6$)-alkenylgruppe oder eine ($C_1$ bis $C_6$)-Alkoxycarbonyl-($C_1$ bis $C_6$)-alkylgruppe,

mit der Maßgabe, dass in Formel (II) $R^1$ und $R^2$ nicht gleichzeitig für ein Wasserstoffatom stehen.

Falls die Verbindungen gemäß Formel (I) mindestens ein Chiralitätszentum enthalten, sind selbstverständlich alle Stereoisomere allein sowie deren Mischungen, insbesondere deren Racemate, erfindungsgemäß.

Die Verbindungen gemäß Formel (I) können neben der Form als freie Säure oder als dessen Salz auch als inneres Salz vorliegen, insbesondere dann, wenn neben der Sulfinat-Gruppe der Formel (I) zusätzlich ein kationischer Substituent (siehe Definition $R^2$ und $R^7$) im Molekül enthalten ist.

Wenn die Verbindung der Formel (I) als Säure vorliegt, bedeuten die Reste M, M', M" und/oder $M^{III}$ ein Wasserstoffatom. Die Fragmente MO- der Formel (I), M'O- der Formel (III) und $M^{II}$O-bzw. $M^{III}$O- gemäß $R^1$ bzw. $R^2$ aus Formel (II) bilden in diesem Fall eine Hydroxygruppe. Wenn die Sulfinsäure der Formel (I) als Salz vorliegt, steht mindestens einer der Reste M, M', $M^{II}$ oder $M^{III}$ für ein Äquivalent eines ein- oder mehrwertigen Kations. Das ein oder mehrwertige Kation $M^{z+}$ mit einer Ladungszahl z von eins oder höher dient lediglich aus Gründen der Elektroneutralität zur Kompensation der einfach negativen Ladung des bei Salzbildung vorliegenden Sulfinat-Fragments

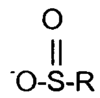

aus Formel (I) bzw. mutatis mutandis aus Formel (III), bzw. der Carboxylat-Fragmente der Reste aus $R^1$ bzw. $R^2$ der Formel (II). Das dafür zu verwendende Äquivalent des entsprechenden Kations beträgt 1/z. Das Fragment MO- der Formel (I) bzw. das Fragment M'O- der Formel (III) stehen im Fall der Salzbildung für die Gruppe: 1/z ($M^{z+}$) -O- bzw. die Gruppe: 1/z ($M'^{z+}$) -O- Gleiches gilt mutatis mutandis für M" und $M^{III}$.

Als entsprechende ein- oder mehrwertige Kationen kommen prinzipiell alle Kationen in Frage, die keine Redox-Reaktion mit dem übrigen Sulfinat-Fragment der Formel (I) eingehen. Insbesondere sind dies Metallkationen der physiologisch verträglichen Metalle aus den Gruppen la, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (I) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M, M', M" und $M^{III}$ stehen bevorzugt unabhängig voneinander für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Das Äquivalent des gegebenenfalls vorhandenen ein- oder mehrwertigen Anions gemäß Formel (I) wird, analog zur Definition der Kationäquivalente, zur Wahrung der Elektroneutralität durch Formulierung eines stöchiometrischen Koeffizienten kleiner 1 vor der Bezeichnung des Anions beschrieben. Die besagten Anionen werden im Weiteren definitionsgemäß mit $An^-$ symbolisiert. Sie werden bevorzugt ausgewählt aus Halogenid, ½ Sulfat, Hydrogensulfat, ½ Carbonat, Hydrogencarbonat, 1/3 Phosphat, ½ Hydrogenphosphat, Dihydrogenphosphat oder p-Toluolsulfonat. Besonders bevorzugt steht das Anion für Chlorid, Bromid, p-Toluolsulfonat oder Hydrogensulfat.

Erfindungsgemäß sind weiterhin solche Sulfinsäurederivate gemäß Formel (I) bevorzugt, in denen die Reste Y der Formel (II) bzw. Formel (III) und Y' der Formel (III) unabhängig voneinander eine Hydroxygruppe oder eine Gruppe $-NH_2$ bedeuten.

Sulfinsäurederivate, in denen $R^1$ für ein Wasserstoffatom oder eine Methylgruppe steht, sind erfindungsgemäß bevorzugt.

Der Rest R gemäß Formel (I) muß zur Lösung der technischen Aufgabe für einen der oben beschriebenen Reste stehen, wobei es bevorzugt ist, dass R für einen Rest der oben genannten Formeln (II) bis (VI) steht.

Steht der Rest R der Formel (I) für einen Rest der oben genannten Formel (II), dann ergibt sich als bevorzugte erste Ausführungsform aus der Formel (I) das erfindungsgemäße Sulfinsäurederivat der Formel (Ia),

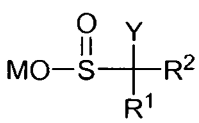

(Ia)

worin M, Y, $R^1$ und $R^2$ die unter den Formeln (I) und (II) definierte Bedeutung haben. Die zuvor erwähnten bevorzugten Definitionen der Reste M, Y und $R^1$ gelten auch hier, allein oder gemeinsam auf Formel (Ia) angewendet, als bevorzugt.

Es ist erfindungsgemäß, wenn gemäß einer bevorzugten Ausführungsform der Erfindung der Rest $R^2$ gemäß Formel (II) bzw. Formel (Ia) für einen aliphatischen oder aromatischen Heterozyklus steht, welcher gegebenenfalls

substituiert sein kann. Falls die oben genannten ausgewählten aliphatischen oder aromatischen Heterozyklen dieser Ausführungsform substituiert sind, dann sind diese bevorzugt mit mindestens einem Rest aus ($C_1$ bis $C_6$)-Alkyl, ($C_2$ bis $C_6$)-Alkenyl, ($C_1$ bis $C_6$)-Hydroxyalkyl, Aryl-($C_1$ bis $C_6$)-alkyl, Hydroxy, ($C_1$ bis $C_6$)-Alkoxy, Amino, Di($C_1$ bis $C_6$)alkylamino, Nitro, Halogen, Carbamoyl, Sulfonamido, Cyano oder Carboxamido, substituiert.

Im Rahmen dieser Ausführungsform ist es wiederum bevorzugt, die aliphatischen oder aromatischen Heterozyklen des Rests $R^2$ auszuwählen aus Thienyl, Furyl, Pyrrolyl, Imidazolyl, Thiazolyl, Oxazolyl, Pyrazolyl, Pyridyl, Pyrimidinyl, Pyrazyl, Pyridazyl, Benzimidazolyl, Benzothiazolyl, Benzoxazolyl, Indolyl, Chinolinyl, Chinoxalinyl oder Chinazolinyl, welche gegebenenfalls substituiert sein können, bevorzugt mit den oben genannten Substituenten, besonders bevorzugt mit mindestens einem Rest aus ($C_1$ bis $C_6$)-Alkyl, ($C_1$ bis $C_6$)-Hydroxyalkyl, Hydroxy, Amino, ($C_1$ bis $C_6$)-Alkoxy, Carboxy, Halogenatom, Nitrogruppe oder Sulfonsäurerest.

Dabei sind besonders bevorzugte Sulfinsäurederivate dieser Ausführungsform Verbindungen der nachfolgenden Formeln (Ia-1) bis (Ia-4)

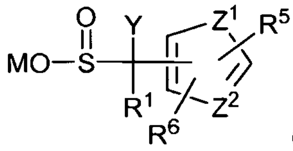
(Ia-1)

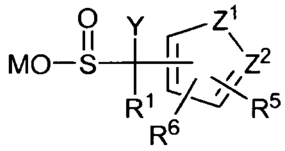
(Ia-2)

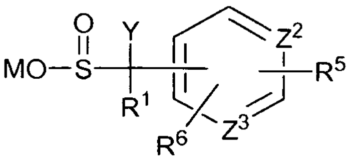
(Ia-3)

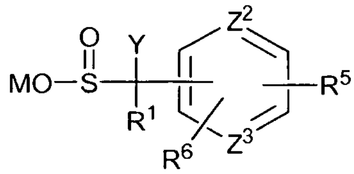
(Ia-4)

worin

| | |
|---|---|
| M, Y und $R^1$ | die unter Formel (I) definierten Bedeutungen haben, |
| $Z^1$ | für ein Sauerstoffatom, ein Schwefelatom oder eine Gruppe N-$R^{IV}$ steht, worin $R^{IV}$ ein Wasserstoffatom, eine ($C_1$ bis $C_6$)-Alkylgruppe, eine ($C_2$ bis $C_6$)-Alkenylgruppe, eine ($C_1$ bis $C_6$)-Hydroxyalkylgruppe oder eine Aryl-($C_1$ bis $C_6$)-alkylgruppe bedeutet, |
| $Z^2$ und $Z^3$ $R^5$ und $R^6$ | unabhängig voneinander für eine Gruppe CH oder für ein Stickstoffatom stehen, unabhängig voneinander stehen für ein Wasserstoffatom, eine ($C_1$ bis $C_6$)-Alkylgruppe, eine ($C_2$ bis $C_6$)-Alkenylgruppe, eine ($C_1$ bis $C_6$)-Hydroxyalkylgruppe, eine Hydroxygruppe, eine ($C_1$ bis $C_6$)-Alkoxygruppe, eine Aminogruppe, eine Di($C_1$ bis $C_6$)alkylaminogruppe, eine Nitrogruppe, eine Halogenatom, eine Carbamoylgruppe, eine Sulfonamidogruppe, eine Cyanogruppe oder eine Carboxamidogruppe oder gemeinsam eine Benzanellierung bilden, welche wiederum substituiert sein kann. |

Die Gruppe N-$R^{IV}$ aus Rest $Z^1$ bildet im Ring des Heterozyklus eine Azandiylgruppe -$NR^{IV}$-. Die Gruppe CH der Reste $Z^2$ bzw. $Z^3$ bildet im Ring des Heterozyklus eine Methanylylidengruppe =CH-, die selbstverständlich auch mit einem der Reste $R^5$ bzw. $R^6$ substituiert sein kann.

Wenn in den Formeln (Ia-1) bzw. (Ia-2) $Z^1$ für ein Sauerstoffatom, $Z^2$ für eine Gruppe CH und Y für eine Hydroxygruppe steht, so ist es erfindungsgemäß bevorzugt, wenn in den Formeln (Ia-1) bzw. (Ia-2) $R^5$ und $R^6$ nicht gleichzeitig für ein Wasserstoffatom stehen.

Die zuvor erwähnten bevorzugten Definitionen der Reste M, Y und $R^1$ gelten auch hier, allein oder gemeinsam, auf Formeln (Ia-1) bis (Ia-4) angewendet, als bevorzugt.

Die Benzanellierung aus den Resten $R^5$ und $R^6$ der Formel (Ia-1) bis (Ia-4) ist, wenn sie substituiert ist, bevorzugt mit mindestens einem Rest aus ($C_1$ bis $C_6$)-Alkyl, ($C_2$ bis $C_6$)-Alkenyl, ($C_1$ bis $C_6$)-Hydroxyalkyl Aryl-($C_1$ bis $C_6$)-alkyl, Hydroxy, ($C_1$ bis $C_6$)-Alkoxy, Amino, Di($C_1$ bis $C_6$)alkylamino, Nitro, Halogen, Carbamoyl, Sulfonamido, Cyano oder Carboxamido substituiert.

$R^5$ und $R^6$ gemäß Formeln (Ia-1) bis (Ia-4) stehen besonders bevorzugt für ein Wasserstoffatom, eine Hydroxygruppe, eine ($C_1$ bis $C_6$)-Alkylgruppe, eine ($C_1$ bis $C_6$)-Alkoxygruppe, eine ($C_1$ bis $C_6$)-Hydroxyalkylgruppe, Halogenatom oder Nitrogruppe.

Wenn die Reste $R^1$ und $R^2$ gemäß Formel (II) im Rahmen der ersten Ausführungsform gemeinsam mit dem Restmolekül einen aliphatischen 5-, 6-, oder 7-gliederigen Ring bilden, welcher mindestens ein kationisches, quaterniertes Stickstoffatom als Heteroatom enthält wobei die kationische Ladung gegebenenfalls durch die ein Äquivalent eines ein oder mehrwertigen Anions kompensiert wird, sind folgende Verbindungen gemäß Formeln (Ia-5) und (Ia-6) bevorzugt,

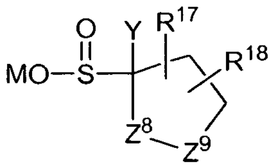
(Ia-5)

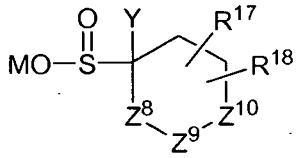
(Ia-6)

worin

| | |
|---|---|
| Y | die unter Formel (I) beschriebenen Definitionen bedeutet, |
| M | die unter Formel (I) beschriebenen Definitionen oder eine negative Ladung bedeutet, |
| $Z^8$, $Z^9$ und $Z^{10}$ | einer dieser Reste eine Azoniumdiyl-Gruppe $N^+R^VR^{VI}$ bedeutet mit $R^V$ und $R^{VI}$ stehen unabhängig voneinander für eine ($C_1$ bis $C_6$)-Alkylgruppe, eine ($C_2$ bis $C_6$)-Alkenylgruppe, eine ($C_1$ bis $C_6$)-Hydroxyalkylgruppe oder eine Aryl-($C_1$ bis $C_6$)-alkylgruppe, und die übrigen dieser Reste für eine $CH_2$-Gruppe stehen, |
| $R^{17}$ und $R^{18}$ | unabhängig voneinander ein Wasserstoffatom, eine ($C_1$ bis $C_6$)-Alkylgruppe, eine Hydroxygruppe, ein Halogenatom oder eine Carboxygruppe bedeuten, |

mit der Maßgabe, dass ein Äquivalent eines ein- oder mehrwertigen Anions zugegen ist, wenn M keine negative Ladung bedeutet.

Die $CH_2$-Gruppe bildet im Ring des Heterozyklus ein Methandiylfragment $-CH_2-$, das selbstverständlich auch mit einem der Reste $R^{17}$ bzw. $R^{18}$ substituiert sein kann.

Die zuvor erwähnten bevorzugten Definitionen der Reste M und Y gelten auch hier, allein oder gemeinsam, auf Formeln (Ia-5) und (Ia-6) angewendet, als bevorzugt. Die unter der Maßgabe beschriebene Bedingung beschreibt die Ausbildung eines inneren Salzes, das ebenso eine bevorzugte Ausführungsform darstellt.

Wenn der Rest $R^2$ der Formel (II) für einen Rest der oben genannten Formel (IV) steht, dann ergibt sich aus der Formel (I) das erfindungsgemäße Sulfinsäurederivat gemäß Formel (Ia-7),

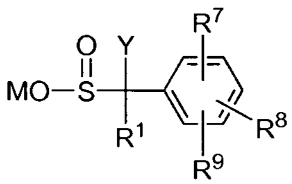
(Ia-7)

worin,
M, Y, $R^1$, $R^7$, $R^8$ und $R^9$ die unter Formel (I) genannten Bedeutungen haben.

Es sind solche Verbindungen der Formel (Ia-7) erfindungsgemäß bevorzugt, bei welchen der Rest $R^7$ eine Carboxygruppe, eine Sulfonsäuregruppe oder eine Gruppe $-N^+R^IR^{II}R^{III}$, mit $R^I$, $R^{II}$ und $R^{III}$ stehen unabhängig voneinander für eine ($C_1$ bis $C_6$)-Alkylgruppe, eine ($C_2$ bis $C_6$)-Alkenylgruppe, eine Aryl($C_1$ bis $C_6$)-alkylgruppe oder eine ($C_1$ bis $C_6$)-Hydroxyalkylgruppe, bedeutet und die Reste $R^8$ und $R^9$ für ein Wasserstoffatom stehen.

Weiterhin erfindungsgemäß bevorzugt eingesetzte Verbindungen der Formel (I) sind Verbindungen der Formel (Ia-8)

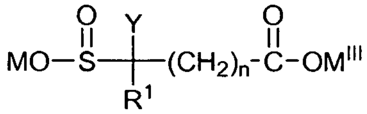
(Ia-8)

worin M, Y, $R^1$, n und $M^{III}$ die unter Formel (I) und (II) genannten Bedeutungen haben. Die zuvor erwähnten bevorzugten Definitionen der Reste M, Y, $R^1$, n, und $M^{III}$ gelten auch hier, allein oder gemeinsam auf Formel (Ia-8) angewendet, als

bevorzugt.

Es sind darüber hinaus solche Verbindungen der Formel (Ia-8) erfindungsgemäß bevorzugt, bei welchen Y eine Hydroxy- oder Aminogruppe bedeutet.

Bevorzugt steht n in Formel (Ia-8) für eine Zahl 0, 1 oder 2.

Steht der Rest R der Formel (I) für einen Rest der oben genannten Formel (III), dann ergibt sich aus der Formel (I) als bevorzugte zweite Ausführungsform der Erfindung das erfindungsgemäße Sulfinsäurederivat der Formel (Ib),

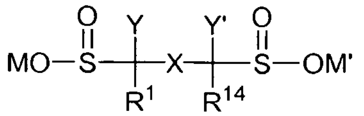

(Ib)

worin M, M', Y, Y', X, $R^1$ und $R^{14}$ die unter den Formeln (I) und (III) definierte Bedeutung haben. Die zuvor erwähnten bevorzugten Definitionen der Reste M, M', Y, Y', X, $R^1$ und $R^{14}$ gelten auch hier, allein oder gemeinsam auf Formel (Ib) angewendet, als bevorzugt.

Der Rest X steht bevorzugt für einen organischen Rest mit zwei freien Valenzen. Als erfindungsgemäße Reste eignen sich prinzipiell alle Organodiyl-Reste, wie z.B. aliphatische oder alizyklische, aromatische oder heteroaromatische Diyl-Reste. Der besagte organische Rest mit zwei freien Valenzen X gemäß Formel (III) bzw. Formel (Ib) wird bevorzugt aus der Gruppe ausgewählt, die gebildet wird aus gegebenenfalls substituierten Resten aus Arendiyl, Heteroarendiyl, Alkandiyl, Alkendiyl, Cycloalkandiyl, Cycloalkendiyl und Di(($C_1$ bis $C_6$)alkylen)-substituierten carbozyklischen oder heterozyklischen Gruppen, die aliphatisch oder aromatisch sind. Falls die oben genannten ausgewählten Reste dieser Ausführungsform substituiert sind, dann sind diese bevorzugt mit mindestens einem Rest aus ($C_1$ bis $C_6$)-Alkyl, ($C_2$ bis $C_6$)-Alkenyl, ($C_1$ bis $C_6$)-Hydroxyalkyl Aryl-($C_1$ bis $C_6$)-alkyl, Hydroxy, ($C_1$ bis $C_6$)-Alkoxy, Amino, Di($C_1$ bis $C_6$)alkylamino, Nitro, Halogen, Carbamoyl, Sulfonamido, Cyano oder Carboxamido substituiert.

Es ist erfindungsgemäß besonders bevorzugt, wenn der Rest X der Formel (III) bzw. der Formel (Ib) für einen organischen Rest mit zwei freien Valenzen steht, der aus einer ($C_1$ bis $C_6$)-Alkandiylgruppe oder aus Resten der Formeln (VII) bis (XI) ausgewählt wird,

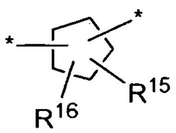

(VII)

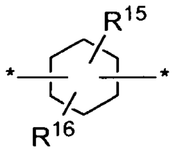

(VIII)

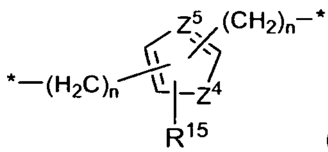

(IX)

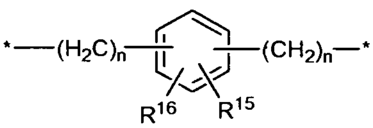

(X)

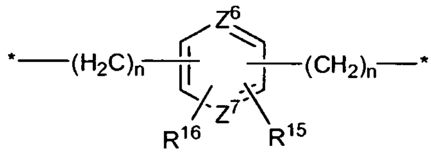

(XI)

worin bedeuten

| | |
|---|---|
| $R^{15}$ und $R^{16}$ | unabhängig voneinander für ein Wasserstoffatom, ein Halogenatom, eine ($C_1$ bis $C_6$)-Alkylgruppe oder eine Carboxygruppe, |
| n | eine ganze Zahl von 0 bis 6, |
| $Z^4$ | eine Gruppe $CH_2$, ein Sauerstoffatom, ein Schwefelatom oder eine Gruppe NR', mit R' = Wasserstoffatom, eine ($C_1$ bis $C_6$)-Alkylgruppe, eine ($C_1$ bis $C_6$)-Hydroxyalkylgruppe oder eine ($C_2$ bis $C_6$)-Polyhydroxyalkylgruppe, |
| $Z^5$, $Z^6$ und $Z^7$ | unabhängig voneinander eine Gruppe CH oder ein Stickstoffatom. |

Die jeweiligen Gruppen CH bzw. $CH_2$ der Reste $Z^4$, $Z^5$, $Z^6$ oder $Z^7$ können selbstverständlich erfindungsgemäß ebenso mit allen erfindungsgemäß im Rahmen der Definition der betroffenen Formel aus den Formeln (VII) bis (XI) möglichen Substituenten substituiert sein.

Falls der organische Rest mit zwei freien Valenzen X aus den Formeln (VII) und (VIII) ausgewählt wird, sind die Reste 2-Chlor-cyclopentan-1,3-diyl, 2-Brom-cyclopentan-1,3-diyl, 2-Chlor-cyclohexan-1,3-diyl und 2-Brom-cyclohexan-1,3-diyl bevorzugte Vertreter.

Steht der Rest R der Formel (I) für einen Rest der oben genannten Formel (IV), dann ergibt sich aus der Formel (I) das erfindungsgemäße Sulfinsäurederivat der dritten Ausführungsform gemäß Formel (Ic),

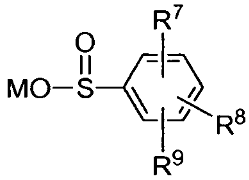

(Ic)

worin M, $R^7$, $R^8$ und $R^9$ die unter den Formeln (I) und (IV) definierte Bedeutung haben. Die zuvor erwähnten bevorzugten Definitionen der Reste M, $R^7$, $R^8$ und $R^9$ gelten auch hier, allein oder gemeinsam auf Formel (Ic) angewendet, als bevorzugt.

Besonders bevorzugt sind Reste R gemäß Formel (IV), die ausgewählt werden, aus 4-Cyanophenyl, 4-Carboxyphenyl, 4-Carboxymethyloxy und 4-Trimethylammoniophenyl.

Steht der Rest R der Formel (I) für einen Rest der oben genannten Formel (V), dann ergibt sich aus der Formel (I) das erfindungsgemäße Sulfinsäurederivat der vierten Ausführungsform gemäß Formel (Id),

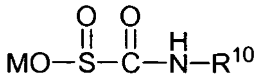

(Id)

worin,

M und $R^{10}$ die unter Formel (I) genannten Bedeutungen haben. Die zuvor erwähnten bevorzugten Definitionen der Reste M und $R^{10}$ gelten auch hier, allein oder gemeinsam auf Formel (Id) angewendet, als bevorzugt.

Steht der Rest R der Formel (I) für einen Rest der oben genannten Formel (VI), dann ergibt sich aus der Formel (I) das erfindungsgemäße Sulfinsäurederivat der fünften Ausführungsform gemäß Formel (Ie),

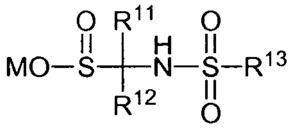

(Ie)

worin,

M, $R^{11}$, $R^{12}$ und $R^{13}$ die unter Formel (I) genannten Bedeutungen haben. Die zuvor erwähnten bevorzugten Definitionen der Reste M, $R^{11}$, $R^{12}$ und $R^{13}$ gelten auch hier, allein oder gemeinsam auf Formel (Ie) angewendet, als bevorzugt.

Im Folgenden sollen Beispiele für die als Substituenten im Rahmen dieser Anmeldung genannten Gruppen bzw. Reste erwähnt werden. Beispiele für ($C_1$ bis $C_6$)-Alkylreste sind lineare, verzweigte oder zyklische ($C_1$ bis $C_6$)-Alkylgruppen, wobei lieneare oder verzweigte ($C_1$ bis $C_6$)-Alkylgruppen bevorzugt sind. Insbesondere sind die Gruppen Methyl, Ethyl, Propyl, Isopropyl, n-Butyl, sec-Butyl, tert.-Butyl, n-Pentyl und n-Hexyl geeignet. Beispiele für entsprechend geeignete zyklische Alkylgruppen sind Cyclopentyl und Cyclohexyl. Beispiele für bevorzugte ($C_2$ bis $C_6$)-Alkenylreste sind Vinyl, Allyl und Butenyl. Erfindungsgemäß bevorzugte ($C_1$ bis $C_6$)-Alkoxyreste sind beispielsweise eine Methoxy- oder eine Ethoxygruppe. Die Methoxycarbonyl-, Ethoxycarbonyl-, n-Propoxycarbonyl-, Isopropoxycarbonyl-, n-Butoxycarbonyl-, sec-Butoxycarbonyl- und tert-Butoxycarbonylgruppe sind Beispiele für ($C_1$ bis $C_6$)-Alkoxycarbonylgruppen; die Methoxycarbonyl- und die Ethoxycarbonylgruppe sind dabei besonders bevorzugt.

Die 2-Methoxycarbonylethyl-, 2-Ethoxycarbonylethyl-, 2-Propoxycarbonylethyl-, 2-Isopropoxycarbonylethyl-, 2-Butoxycarbonylethyl-, 2-sec-Butoxycarbonylethyl-, 2-tert-Butoxycarbonylethyl-, 3-Methoxycarbonylpropyl-, 3-Ethoxycarbonylpropyl-, 3-Propoxycarbonylpropyl-, 3-Isopropoxycarbonylpropyl-, 3-Butoxycarbonylpropyl-, 3-sec-Butoxycarbonylpropyl- und 3-tert-Butoxycarbonylpropylgruppe sind Beispiele für ($C_1$ bis $C_6$)-Alkoxycarbonyl-($C_1$ bis $C_6$)-alkylgruppen.

Beispiele für erfindungsgemäße Cyano-($C_1$ bis $C_6$)-alkylgruppen sind Cyanomethyl, 2-Cyanoethyl, 3-Cyanopropyl, 4-Cyanobutyl und 5-Cyanopentyl.

Weiterhin können als bevorzugte Beispiele für eine ($C_1$ bis $C_6$)-Monohydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe genannt werden. Eine 2-Hydroxyethylgruppe ist besonders bevorzugt. Beispiele für eine ($C_2$ bis $C_6$)-Polyhydroxyalkylgruppe sind die 2,3-Dihydroxypropylgruppe, 3,4-Dihydroxybutylgruppe und die 2,4-Dihydroxybutylgruppe. Die Methoxyethyl-, Ethoxyethyl-, Methoxypropyl-, Methoxybutyl-, Ethoxybutyl- und die Methoxyhexylgruppe sind Beispiele für erfindungsgemäße ($C_1$ bis $C_6$)-Alkoxy-($C_1$ bis $C_6$)-alkylgruppen.
Eine bevorzugte Hydroxy-($C_1$ bis $C_6$)-alkoxygruppe ist die 2-Hydroxyethoxygruppe. Bevorzugte Arylgruppen sind Phenyl, Naphthyl und Biphenyl.
Bevorzugte Heteroarylgruppen sind Thienyl, Furyl, Pyrrolyl, Imidazolyl, Thiazolyl, Oxazolyl, Pyrazolyl, Pyridyl, Pyrimidinyl, Pyrazyl, Pyridazyl, Benzimidazolyl, Benzothiazolyl, Benzoxazolyl, Indolyl, Chinolinyl, Chinoxalinyl und Chinazolinyl. Die Trifluomethyl- und die Pentafluorethylgruppe sind bevorzugte Perfluor-($C_1$ bis $C_6$)-alkylgruppen.
Beispiele für Halogenatome sind F-, Cl-, Br- oder I-Atome, wobei Cl- und Br-Atome ganz besonders bevorzugt sind. Bevorzugte Aryl-($C_1$ bis $C_6$)-alkylgruppen sind Benzyl und 2-Phenylethyl. Die Trimethylammonium- und Diethylmethylammonium- sind Beispiele für eine Gruppe - $N^+R^IR^{II}R^{III}$.
Die 2-Trimethylammoniumethyl- ist ein Beispiel für eine N,N,N-Tri[($C_1$ bis $C_6$)-akyl]ammonium-($C_1$ bis $C_6$)-alkylgruppe. Eine bevorzugte ($C_1$ bis $C_6$)-Carboxyalkylgruppe ist die 3-Carboxypropylgruppe. Die weiteren verwendeten Begriffe leiten sich erfindungsgemäß von den hier gegebenen Definitionen ab.

Ganz besonders bevorzugte Vertreter der Sulfinsäurederivate gemäß Formel (I) sind die Sulfinsäuren gemäß folgender Liste oder deren Salze mit einem Äquivalent mindestens eines ein- oder mehrwertigen Kations:

| Name: | korrespondierende Struktur: |
|---|---|
| 2-Furyl(amino)methansulfinsäure, | 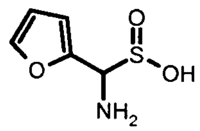 |
| Amino(thien-2-yl)methansulfinsäure, | 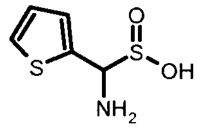 |
| Amino(1*H*-imidazol-2-yl)methansulfinsäure, | 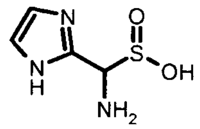 |
| Amino(1,3-thiazol-2-yl)methansulfinsäure, | 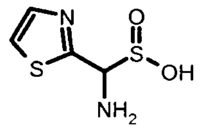 |
| Amino(1,3-oxazol-2-yl)methansulfinsäure, | 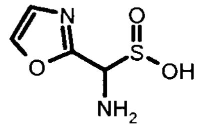 |
| Amino(1H-pyrrol-2-yl)methansulfinsäure, | 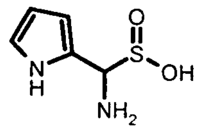 |
| Amino(hydroxyl-2-yl)methansulfinsäure, | 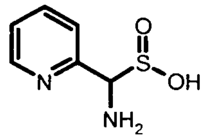 |
| Amino(hydroxyl-4-yl)methansulfinsäure, | 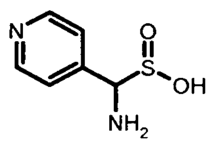 |
| Amino[3-hydroxy-5-(hydroxymethyl)-2-methylpyridin-4-yl]methansulfinsäure, | 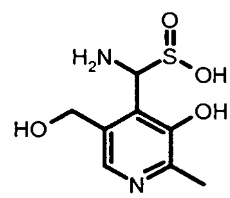 |

(fortgesetzt)

Amino(chinolin-2-yl)methansulfinsäure,

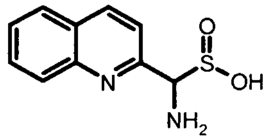

Amino(chinolin-4-yl)methansulfinsäure,

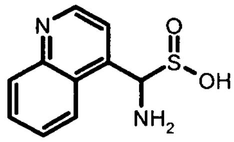

1*H*-Benzimidazol-2-yl(amino)methansulfinsäure,

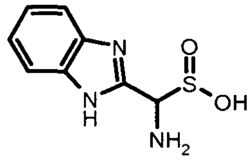

1,3-Benzothiazol-2-yl(amino)methansulfinsäure,

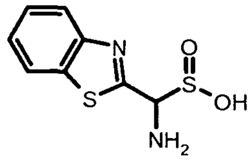

1,3-Benzoxazol-2-yl(amino)methansulfinsäure,

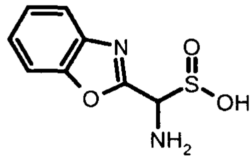

Hydroxy(thien-2-yl)methansulfinsäure,

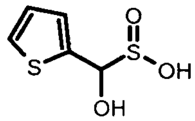

Hydroxy(thien-3-yl)methansulfinsäure

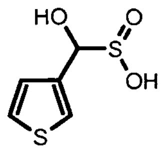

(3-Bromthien-2-yl)(hydroxyl)methansulfinsäure,

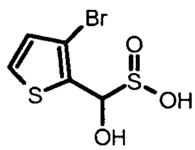

Hydroxy(1H-imidazol-2-yl)methansulfinsäure,

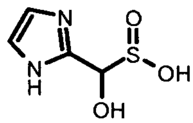

Hydroxy(1H-imidazol-5-yl)methansulfinsäure,

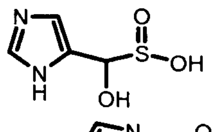

Hydroxy(1-methyl-5-nitro-1*H*-imidazol-2-yl)methansulfinsäure,

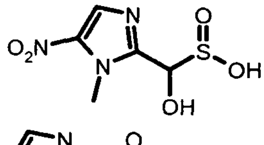

Hydroxy(1,3-thiazol-2-yl)methansulfinsäure,

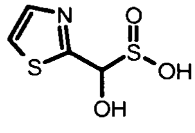

(fortgesetzt)

Hydroxy(1,3-oxazol-2-yl)methansulfinsäure,

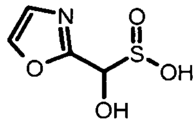

Hydroxy(1*H*-pyrrol-2-yl)methansulfinsäure,

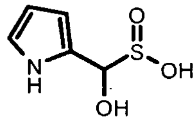

Hydroxy(hydroxyl-2-yl)methansulfinsäure,

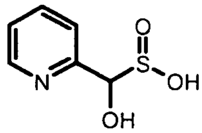

Hydroxy(hydroxyl-4-yl)methansulfinsäure,

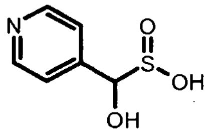

Hydroxy[3-hydroxy-5-(hydroxymethyl)-2-methylpyridin-4-yl]-methansulfinsäure,

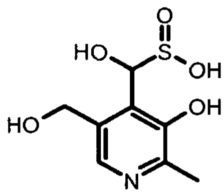

Hydroxy(chinolin-2-yl)methansulfinsäure,

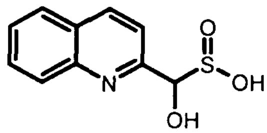

Hydroxy(chinolin-4-yl)methansulfinsäure,

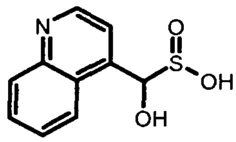

1*H*-Benzimidazol-2-yl(hydroxyl)methansulfinsäure,

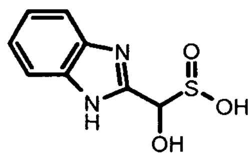

1,3-Benzothiazol-2-yl(hydroxyl)methansulfinsäure,

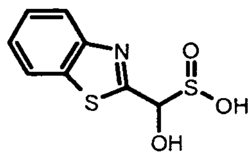

1,3-Benzoxazol-2-yl(hydroxy)methansulfinsäure,

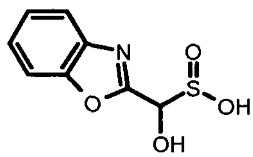

1,2-Dihydroxyethan-1,2-disulfinsäure,

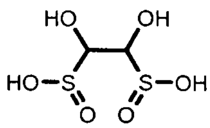

1,3-Dihydroxypropan-1,3-disulfinsäure,

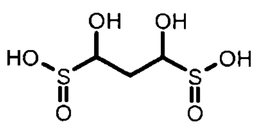

(fortgesetzt)

Hydroxy{4-[hydroxy(sulfino)methyl]phenyl}methan-sulfinsäure,

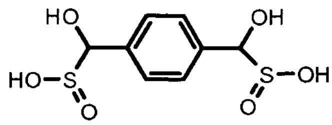

{2-Chlor-3-[hydroxy(sulfino)methyl]cyclohexyl}-(hydroxy)methansulfinsäure,

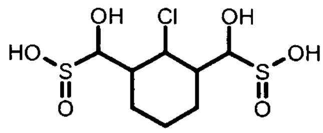

{2-Chlor-3-[hydroxyl(sulfino)methyl]cyclopentyl}-(hydroxyl)methansulfinsäure,

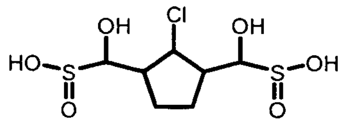

Hydroxy{5-[hydroxy(sulfino)methyl]thien-2-yl}methansulfinsäure,

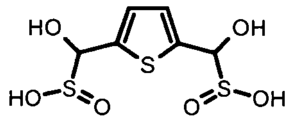

Hydroxy{2-[hydroxy(sulfino)methyl]phenyl}methan-sulfinsäure,

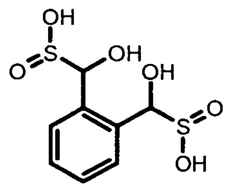

Hydroxy{3-[hydroxy(sulfino)methyl]phenyl}methan-sulfinsäure,

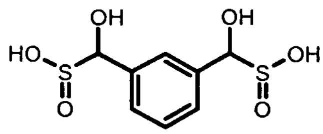

1,5-Dihydroxypentan-1,5-disulfinsäure,

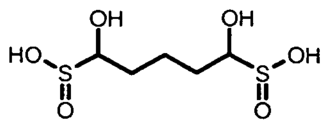

4-Hydroxy-4-sulfinobutansäure,

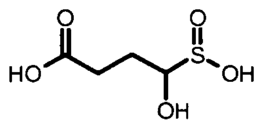

1-Hydroxy-4-methoxy-4-oxobutan-1-sulfinsäure,

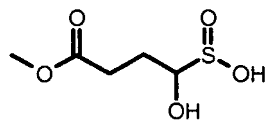

1-Hydroxy-4-ethoxy-4-oxobutan-1-sulfinsäure,

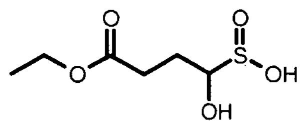

4-Hydroxy-4-sulfinopentansäure,

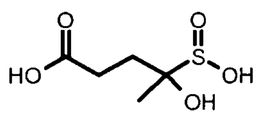

2-Hydroxy-5-methoxy-5-oxopentan-2-sulfinsäure,

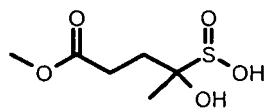

2-Hydroxy-5-ethoxy-5-oxopentan-2-sulfinsäure,

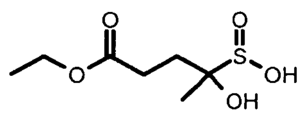

(fortgesetzt)

4-Hydroxy-4-sulfinobut-2-ensäure,

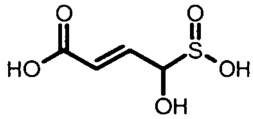

1-Hydroxy-4-methoxy-4-oxobut-2-en-1-sulfinsäure,

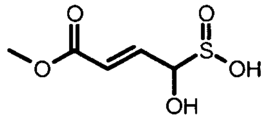

1-Hydroxy-4-ethoxy-4-oxobut-2-en-1-sulfinsäure,

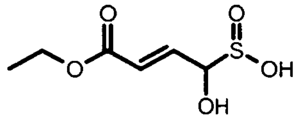

4-Hydroxy-4-sulfinopent-2-ensäure,

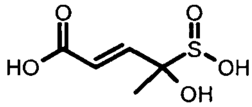

2-Hydroxy-5-methoxy-5-oxopent-3-en-2-sulfinsäure,

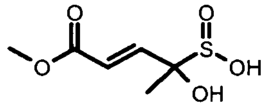

2-Hydroxy-5-ethoxy-5-oxopent-3-en-2-sulfinsäure,

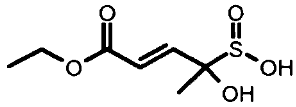

5-Hydroxy-5-sulfinopentansäure,

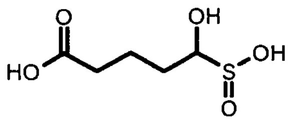

1-Hydroxy-5-methoxy-5-oxopentan-1-sulfinsäure,

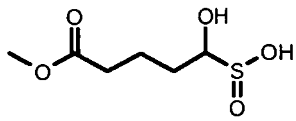

1-Hydroxy-5-ethoxy-5-oxopentan-1-sulfinsäure,

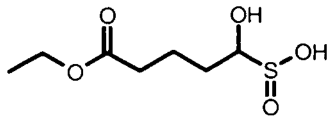

5-Hydroxy-5-sulfinohexansäure,

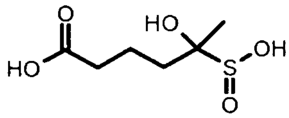

2-Hydroxy-6-methoxy-6-oxohexan-2-sulfinsäure,

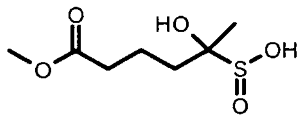

2-Hydroxy-6-ethoxy-6-oxohexan-2-sulfinsäure,

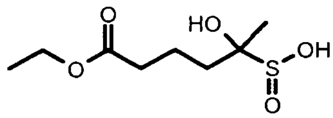

4-[Hydroxy(sulfino)methyl]benzoesäure,

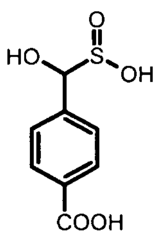

(fortgesetzt)

4-[Amino(sulfino)methyl]benzoesäure,

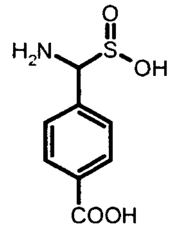

{4-[Hydroxy(sulfino)methyl]phenoxy}-essigsäure,

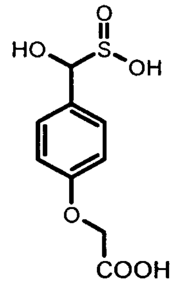

{4-[Amino(sulfino)methyl]phenoxy}-essigsäure,

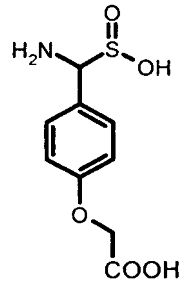

3-Hydroxy-4-[hydroxy(sulfino)methyl]benzoesäure,

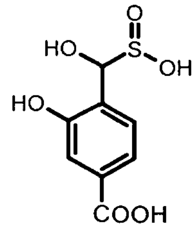

3-Hydroxy-4-[amino(sulfino)methyl]benzoesäure,

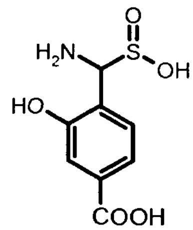

(4-Cyanophenyl)(hydroxy)-methansulfinsäure,

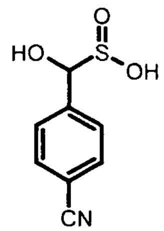

(4-Cyanophenyl)(amino)-methansulfinsäure,

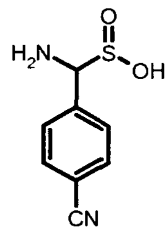

(fortgesetzt)

(4-Nitrophenyl)(hydroxy)-methansulfinsäure,

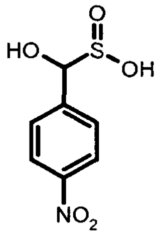

(4-Nitrophenyl)(amino)-methansulfinsäure,

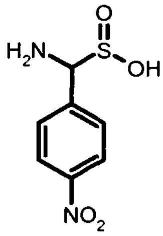

Salz des 4-Hydroxy-1,1-dimethyl-4-sulfinopiperidiniums mit $An^-$ als Äquivalent eines ein- oder mehrwertigen Anions,

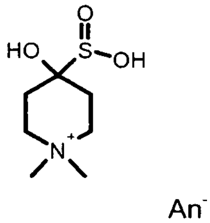

Salz des 1-Allyl-4-hydroxy-1-methyl-4-sulfinopiperidiniums mit $An^-$ als Äquivalent eines ein- oder mehrwertigen Anions,

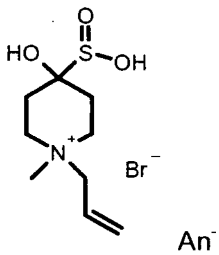

4-Hydroxy-1,1-dimethylpiperidinium-4-sulfinat,

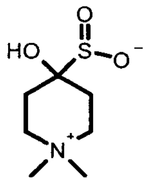

1-Allyl-4-hydroxy-1-methylpiperidinium-4-sulfinat,

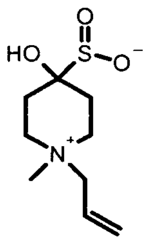

[(2-Methoxy-2-oxoethyl)amino](oxo)methansulfinsäure,

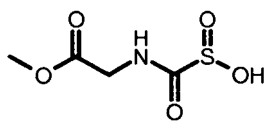

[(Methylsulfonyl)amino]methansulfinsäure,

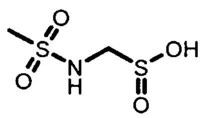

[(Trifluorethylsulfonyl)amino]methansulfinsäure,

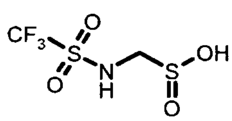

(fortgesetzt)

[(Phenylsulfonyl)amino]methansulfinsäure

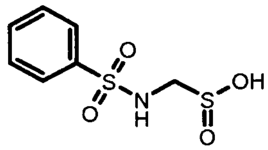

1-Hydroxy-2-(trimethylammonio)ethansulfinat,

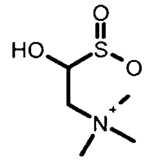

Hydroxy[4-(trimethylammonio)phenyl]methansulfinat,

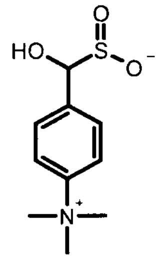

4-[Hydroxy(sulfino)methyl]benzolsulfonsäure und

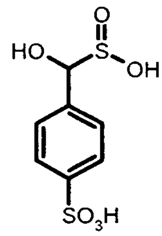

2-[Hydroxy(sulfino)methyl]benzolsulfonsäure

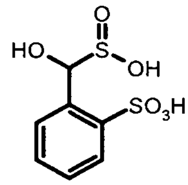

2-Hydroxy-2-sulfino-essigsäure

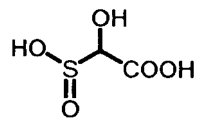

2-Amino-2-sulfino-essigsäure

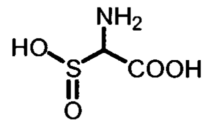

2-Hydroxy-2-sulfino-propionsäure

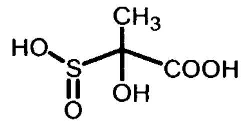

2-Amino-2-sulfino-propionsäure

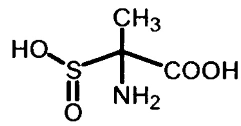

3-Hydroxy-3-sulfinato-propionsäure

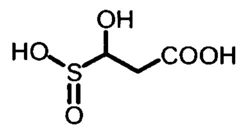

(fortgesetzt)

3-Amino-3-sulfinato-propionsäure

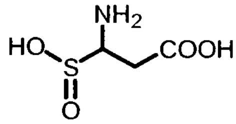

2-Hydroxy-2-sulfinato-essigsäureethylester

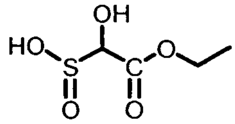

2-Amino-2-sulfinato-essigsäureethylester

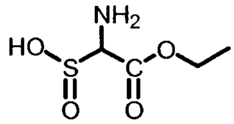

1-Hydroxy-2-methoxy-2-oxoethansulfinsäure

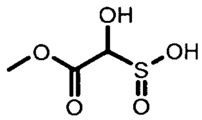

2-Amino-1-hydroxy-2-oxoethansulfinsäure

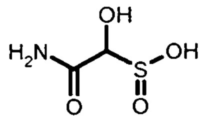

2-(Dimethylamino)-1-hydroxy-2-oxoethansulfinsäure

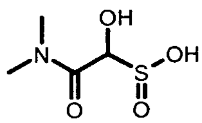

Hydroxy(sulfino)methansulfonsäure

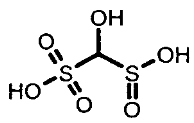

Hydroxy(phenyl)methansulfinsäure

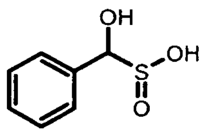

Hydroxy(4-hydroxyphenyl)methansulfinsäure

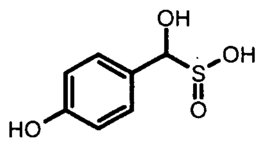

Hydroxy(4-methoxyphenyl)methansulfinsäure

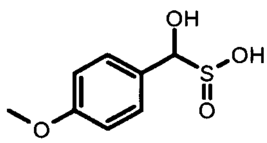

Für die bevorzugten ein- oder mehrwertigen Kationen aller Salze der zuvor genannten Verbindungen gilt das zuvor Gesagte.

Die Herstellung der erfindungsgemäß eingesetzten Sulfinsäuren der Formel (I) erfolgt beispielsweise aus der Umsetzung von Dithionit mit entsprechenden Aldehyden bzw. Ketonen analog zu der Herstellvorschrift gemäß WO-A1-99/18067.

Die erfindungsgemäß einsetzbaren Adsorptionsmittel können hydrophil oder hydrophob sein, wobei hydrophile Adsorptionsmittel bevorzugt sind.

Die erfindungsgemäßen Adsorptionsmittel sind bevorzugt in einer Menge von 2 bis 20 Gew.-%, besonders bevorzugt von 5 bis 10 Gew.-%, jeweils bezogen auf das Gewicht des Mittels, enthalten.

Die erfindungsgemäßen Adsorptionsmittel und die erfindungsgemäßen Reduktionsmittel sind in dem erfindungsgemäßen Mittel bevorzugt in einem Gewichtsverhältnis (Adsorptionsmittel zu Reduktionsmittel) von 1 zu 2 bis 2 zu 1, besonders bevorzugt von 1.2 zu 1 bis 1 zu 1.2 enthalten.

Erfindungsgemäße Adsorptionsmittel besitzen bevorzugt eine spezifische BET-Oberfläche von 40 bis 4000

$m^2/g$, insbesondere von 100 bis 1000 $m^2/g$ (jeweils bestimmt nach DIN ISO 9277: 2003-05).

Bevorzugte erfindungsgemäße Adsorptionsmittel besitzen Poren, insbesondere Mesoporen und/oder Mikroporen und/oder Macroporen. Eine Porengröße von wenigstens 0,4 nm ist eine bevorzugt geeignete Porengröße. Die Adsorptionsmittel weisen bevorzugt ein mittleres Porenvolumen von 0,01 bis 5,0 $cm^3/g$, isnbesondere von 0,1 bis 4 $cm^3/g$, auf.

Erfindungsgemäß verwendbare Adsorptionsmittel werden ausgewählt aus Polymeren und/oder Copolymeren des Vinylpyrrolidons.

Bevorzugt geeignete Polymere des Vinylpyrrolidons sind dessen Homo- und/oder Copolymere. Als erfindungsgemäße Copolymere sind Copolymere mit Vinylacetat bevorzugt verwendbar. Es weiterhin bevorzugt, dass die Polymere des Vinylpyrrolidons kein permanent quarternisiertes Stickstoffatom tragen. Bevorzugte Handelsprodukte sind Luviskol® K 17, Luviskol® K 30, Luviskol® K 80, Luviskol® K 90 (alle Fa. BASF).

In einer erfindungsgemäß bevorzugten Ausführungsform enthalten die erfindungsgemäßen Mittel zusätzlich mindestens eine Verbindung, die mindestens ein quartäres Stickstoffatom besitzt. Besagte Verbindungen, besitzend mindestens ein quartäres Stickstoffatom, sind von den o.g. organischen Sulfinsäuren und den Adsorptionsmitteln verschieden.

Unter Verbindungen, besitzend mindestens ein quartäres Stickstoffatom, verstehen sich im Sinne der Erfindung alle chemischen Verbindungen, die mindestens ein quartäres, kationisches Stickstoffatom tragen. Das quartäre Stickstoffatom ist vierbindig. Somit sind quartäre Ammoniumreste und quartäre Iminiumreste umfasst. Die an das quartäre Stickstoffatom bindenden Gruppen sind allesamt von Wasserstoff verschieden und bilden mindestens drei Bindungen zu Kohlenstoffatomen organischer Reste aus. Diese Verbindungen werden auch als quartäre Ammoniumverbindungen bezeichnet.

Die erfindungsgemäßen Mittel enthalten bevorzugt mindestens eine Verbindung, besitzend mindestens ein quartäres Stickstoffatom, ausgewählt aus der Gruppe, die gebildet wird aus Verbindungen mit einem quartären Stickstoffatom, Verbindungen mit zwei quartären Stickstoffatomen, Verbindungen mit drei quartären Stickstoffatomen, organischen N-Oxiden und Polymeren abgeleitet von einem Monomer, enthaltend mindestens ein quartäres Stickstoffatom. Sowohl im Allgemeinen als auch für alle Vertreter der vorangegangenen Liste ist es bevorzugt, wenn die Verbindungen mit quartärem Stickstoffatom netto kationisch geladen sind. Die besagten Verbindungen liegen in letztgenannter Ausführungsform folglich nicht als ungeladenes Zwitterion oder gar anionisch vor.

Die Verbindungen, besitzend mindestens ein quartäres Stickstoffatom sind in dem erfindungsgemäßen Mittel bevorzugt in einer Menge von 0,01 Gew.-% bis 20 Gew.-%, besonders bevorzugt von 0,1 Gew.-% bis 5 Gew.-%, jeweils bezogen auf das Gewicht des anwendungsbereiten Mittels, enthalten.

Für diese Ausführungsform erfindungsgemäß besonders geeignete Verbindung, besitzend mindestens ein quartäres Stickstoffatom, sind Verbindungen mit der Formel (Q1),

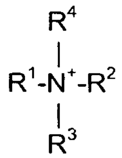

$X^-$ (Q1)

worin bedeuten

$R^1$ und $R^2$ unabhängig voneinander eine lineare oder verzweigte ($C_1$ bis $C_{30}$)-Alkylgrupppe, eine ($C_2$ bis $C_{30}$)-Acylgruppe oder eine Gruppe $-A^1-A^2-A^3-R^5$
worin
$R^5$ eine lineare oder verzweigte ($C_8$ bis $C_{30}$)-Alkylgruppe, eine lineare oder verzweigte ($C_8$ bis $C_{30}$)-Alkenylgruppe, eine Hydroxy-($C_8$- bis $C_{30}$)-alkylgruppe oder eine Oligohydroxy-($C_8$- bis $C_{30}$)-alkylgruppe bedeutet,
$A^1$ eine direkte Bindung oder eine Carbonylgruppe bedeutet,
$A^2$ eine direkte Bindung, eine Gruppe NH oder ein Sauerstoffatom bedeutet,
$A^3$ eine ($C_2$ bis $C_4$)-Alkylengruppe oder eine ($C_2$ bis $C_4$)-Hydroxyalkylengruppe bedeutet

$R^3$ eine lineare oder verzweigte ($C_8$ bis $C_{30}$)-Alkylgruppe, eine ($C_8$ bis $C_{30}$)-Hydroxyalkylgruppe, eine ($C_2$ bis $C_{30}$)-Acylgruppe, eine ($C_2$ bis $C_4$)-Hydroxyalkylgruppe oder eine Aryl-($C_1$ bis $C_6$)-alkylgruppe,

$R^4$ eine ($C_1$ bis $C_4$)-Alkylgruppe oder eine ($C_2$ bis $C_4$)-Hydroxyalkylgruppe,

$X^-$ ein physiologisch verträgliches organisches oder anorganisches Anion.

$R^4$ steht gemäß Formel (Q1) bevorzugt für eine Methyl-, eine Ethyl-, eine Benzyl- oder eine 2-Hydroxyethyl-

gruppe.

Beispiele für eine ($C_1$- bis $C_4$)-Alkylgruppe sind Methyl, Ethyl, Propyl, Isopropyl, n-Butyl, sec-Butyl und tert.-Butyl. Beispiele für eine ($C_8$- bis $C_{30}$)-Alkylgruppe sind n-Octyl, n-Decyl, n-Dodecyl, n-Tetradecyl, n-Hexadecyl, Stearyl, Behenyl, Isostearyl und Eicosyl.
Beispiele für eine ($C_1$- bis $C_{30}$)-Alkylgruppe sind die vorgenannten Beispiele für die jeweiligen Alkylgruppen sowie n-Pentyl, eine Isopentyl, n-Hexyl und eine n-Heptylgruppe.
Beispiele für eine ($C_2$- bis $C_4$)-Alkenylgruppe sind Vinyl, Allyl und Butenyl.
Beispiele für eine ($C_2$- bis $C_4$)-Hydroxyalkylgruppe sind eine 2-Hydroxyethyl-, eine 2-Hydroxypropyl, eine 3-Hydroxypropyl- und eine 4-Hydroxybutylgruppe.
Beispiele für eine ($C_2$- bis $C_4$)-Alkylengruppe ist Ethylen, Propan-1,2-diyl, Propan-1,3-diyl, und Butan-1,4-diyl.
Beispiele für eine (orbis $C_4$)-Hydroxyalkylengruppe ist 2-Hydroxyethylen, 1-Hydroxy-propan-1,2-diyl, 1-Hydroxy-propan-1,3-diyl und 2-Hydroxy-propan-1,3-diyl.
Beispiele für eine ($C_1$-bis $C_4$)-Alkylengruppe sind Methylen, Ethylen, Propan-1,2-diyl, Propan-1,3-diyl, und Butan-1,4-diyl.
Beispiele für ($C_2$- bis $C_{30}$)-Acylgruppen sind Octanoyl, Decanoyl, Dodecanoyl, Tetradecanoyl, Hexadecanoyl, Octadecanoyl und Eicosanoyl.
Beispiele für lineare oder verzweigte ($C_8$-$C_{30}$)-Alkenylgruppen sind 9-Octadecen-1-yl, 9,12-Octadecadien-1-yl, 9,12,15-Octadecatrien-1-yl und 13-Docosen-1-yl.
Hydroxy-($C_8$-$C_{30}$)-alkylgruppen sind beispielsweise 2-Hydroxydecyl, 2-Hydroxydodecyl, 2-Hydroxytetradecyl, 2-Hydroxyhexadecyl und 2-Hydroxyoctadecyl.
Als physiologisch verträgliches organisches oder anorganisches Gegenionen X gemäß Formel (Q1) kommen beispielsweise Halogenidionen, Sulfationen, Phosphationen, Methosulfationen sowie organische Ionen wie Lactat-, Citrat-, Tartrat- und Acetationen in Betracht. Bevorzugt sind Methosulfat und Halogenidionen, insbesondere Bromid und Chlorid.

Bevorzugte Vertreter des Rests $-A^1-A^2-A^3-R^5$ sind erfindungsgemäß die Gruppen

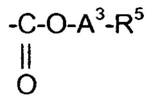 und 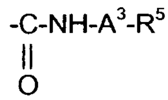

in denen die Gruppen $A^3$ und $R^5$ gemäß Formel (Q1) definiert sind.

Besonders bevorzugte Reste $-A^1-A^2-A^3-R^5$ sind erfindungsgemäß

- eine ω-[($C_8$- bis $C_{30}$)-Alkanoyloxy]-($C_2$- bis $C_4$)-alkylgruppe ($R^5$ = ($C_8$- bis $C_{30}$)-Alkyl, $A^1$ = Carbonyl, $A^2$ = Sauerstoffatom, $A^3$ = ($C_2$- bis $C_4$)-Alkylengruppe),
- eine ω-[($C_8$- bis $C_{30}$)-Alkylamido]-($C_2$- bis $C_4$)-alkylgruppe ($R^5$ = ($C_8$- bis $C_{30}$)-Alkyl, $A^1$ = Carbonyl, $A^2$ = Gruppe NH, $A^3$ = ($C_2$- bis $C_4$)-Alkylengruppe),
- eine ($C_{10}$- bis $C_{34}$)-Hydroxyalkylgruppe (entweder: $R^5$ = ($C_8$- bis $C_{30}$)-Alkyl, $A^1$ = direkte Bindung, $A^2$ = direkte Bindung, $A^3$ = ($C_2$- bis $C_4$)-Hydroxyalkylengruppe oder: $R^5$ = ($C_8$-bis $C_{30}$)-Hydroxyalkyl oder Oligohydroxy-($C_8$- bis $C_{30}$)-alkylgruppe, $A^1$ = direkte Bindung, $A^2$ = direkte Bindung, $A^3$ = ($C_2$- bis $C_4$)-Alkylengruppe.

Beispiele für ω-[($C_8$- bis $C_{30}$)-Alkanoyloxy]-($C_2$- bis $C_4$)-alkylgruppen sind die bevorzugten 2-[($C_8$-bis $C_{30}$)-Acyloxy]ethylgruppen, insbesondere 2-(Dodecanoyloxy)ethyl, 2-(Tetradecanoyloxy)ethyl, 2-(Hexadecanoyloxy)ethyl und 2-(Octadecanoyloxy)ethyl.

Die Verbindungen mit der Formel (Q1) werden bevorzugt ausgewählt aus mindestens einer Verbindung aus der Gruppe, die gebildet wird aus Verbindungen der nachfolgenden Formeln (Q1-a), (Q1-b) und (Q1-c).

Eine der o.g. bevorzugten Gruppen von quartären Ammoniumverbindungen der vorgenannten Formel (Q1) wird definiert aus Verbindungen der Formel (Q1-a)

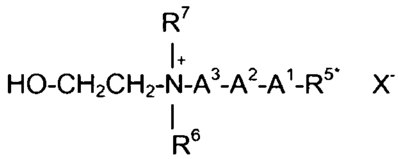

(Q1-a)

worin

- $R^6$ und $R^7$ unabhängig voneinander eine ($C_2$ bis $C_{30}$)-Acylgruppe oder eine lineare oder verzweigte ($C_1$ bis $C_{30}$)-Al-

kylgruppe bedeutet,

- $R^{5*}$ eine lineare oder verzweigte ($C_8$ bis $C_{30}$)-Alkylgruppe oder eine lineare oder verzweigte ($C_8$ bis $C_{30}$)-Alkenylgruppe bedeutet,
- $A^1$ eine direkte Bindung oder eine Carbonylgruppe bedeutet,
- $A^2$ eine direkte Bindung, eine Gruppe NH oder ein Sauerstoffatom bedeutet,
- $A^3$ eine ($C_2$ bis $C_4$)-Alkylengruppe oder eine ($C_2$ bis $C_4$)-Hydroxyalkylengruppe bedeutet und
- $X^-$ ein physiologisch verträgliches organisches oder anorganisches Anion ist,

mit der Maßgabe, daß $A^3$ eine ($C_2$- bis $C_4$)-Hydroxyalkylengruppe bedeutet, wenn $A^1$ und $A^2$ für eine direkte Bindung stehen.

Die andere Gruppe der bevorzugten quartären Ammoniumverbindungen wird ausgewählt aus Verbindungen mit der Formel (Q1-b)

$$R^5\text{-}A^{1*}\text{-}A^{2*}\text{-}A^3\text{-}\overset{\displaystyle R^8}{\overset{|}{N^+}}\underset{\underset{\displaystyle R^9}{|}}{}\text{-}A^3\text{-}A^{2*}\text{-}A^{1*}\text{-}R^5 \quad X^- \qquad \text{(Q1-b)}$$

worin

- $R^8$ und $R^9$ unabhängig voneinander für eine ($C_1$- bis $C_4$)-Alkylgruppe oder ($C_2$- bis $C_4$)-Hydroxyalkylgruppe stehen,
- $R^5$ eine lineare oder verzweigte ($C_8$- bis $C_{30}$)-Alkylgruppe, lineare oder verzweigte ($C_8$-bis $C_{30}$)-Alkenylgruppe, eine Hydroxy-($C_8$- bis $C_{30}$)-alkylgruppe oder eine Oligohydroxy-($C_8$- bis $C_{30}$)-alkylgruppe bedeutet,
- $A^{1*}$ eine Carbonylgruppe bedeutet
- $A^{2*}$ eine Gruppe NH oder ein Sauerstoffatom bedeutet,
- $A^3$ eine ($C_2$- bis $C_4$)-Alkylengruppe, eine ($C_2$- bis $C_4$)-Hydroxyalkylengruppe bedeutet und
- X- ein physiologisch verträgliches organisches oder anorganisches Anion ist.

Es sind solche Verbindungen gemäß Formel (Q1-b) erfindungsgemäß bevorzugt, für die mindestens eine der folgenden Bedingungen gilt:

- $R^8$ und $R^9$ stehen bevorzugt unabhängig voneinander für eine Methylgruppe oder eine 2-Hydroxyethylgruppe.
- wenn $R^8$ und $R^9$ gleich sind, stehen sie bevorzugt für eine ($C_1$- bis $C_4$)-Alkylgruppe, besonders bevorzugt für eine Methylgruppe.
- Es ist bevorzugt, daß $A^3$ ausgewählt wird aus Ethylen, Propan-1,3-diyl, Propan-1,2-diyl, 2-Hydroxypropan-1,3-diyl.

Weitere erfindungsgemäß bevorzugte quaternierte Ammoniumverbindungen der Formel (Q1) werden ausgewählt aus Verbindungen mit der Formel (Q1-c),

$$(R^{10})_y(Me)_{(4-y)}N^+ \, X^- \qquad \text{(Q1-c)}$$

wobei

$R^{10}$ unabhängig voneinander eine ($C_8$- bis $C_{30}$)-Alkylgruppe,
y gleich 1 oder 2 ist und
X- ein physiologisch verträgliches organisches oder anorganisches Anion ist,

In den Verbindungen der Formel (Q1-c) steht der Rest $R^{10}$ bevorzugt für eine Alkylgruppe mit 10 bis 22 Kohlenstoffatomen, besonders bevorzugt mit 10 bis 18 Kohlenstoffatomen.

Die in den erfindungsgemäßen Mitteln dieser Ausführungsform als Verbindungen der Formel (Q1-c) enthaltenen quartären Ammoniumverbindungen sind bevorzugt Halogenide, insbesondere Chloride und Bromide.

Es gelten diejenigen für die Reste gemäß Formel (Q1) genannten Beispiele, als Beispiele für die jeweiligen Reste bzw. Gruppen der Formeln (Q1-a), (Q1-b) und (Q1-c).

Die Verbindungen der Formel (Q1) werden bevorzugt ausgewählt aus der Gruppe, die gebildet wird aus

- Salze, insbesondere Halogenide, des Dicetyldimethylammoniums, Distearyldimethylammoniums (z.B. Dioctadecyldimethylammonium Chlorid als Genamin® DSAc der Firma Clariant), Cetyltrimethylammoniums (z.B. Hexadecyltrimethylammonium Chlorid als Dehyquart® A der Firma Cognis), oder des Stearyltrimethylammoniums,

- quaternierten Estersalzen von Fettsäuren mit Triethanolamin, sogenannte Esterquats, insbesondere den Salzen des N,N-Di(2-(dodecanoyloxy)ethyl)dimethylammoniums, N,N-Di(2-(tetradecanoyloxy)ethyl)dimethylammoniums, N,N-Di(2-(hexadecanoyloxy)ethyl)dimethylammoniums, N,N-Di(2-(hexadecanoyloxy)-propyl)dimethylammoniums, N,N-Di(2-(octadecanoyloxy)ethyl)dimethylammoniums mit einem physiologisch verträglichen, organisch oder anorganischen Anion. Bevorzugte Anionen sind Alkylsulfate, wie z.B. Methylsulfat, und Halogenide, wie Chlorid und Bromid. Solche Produkte werden beispielsweise unter den Warenzeichen Stepantex®, Dehyquart® und Armocare® vertrieben. Die Produkte Armocare® VGH-70, ein N,N-Di(2-Hexadecanoyloxyethyl)dimethylammoniumchlorid, sowie Dehyquart® L80 (INCI-Bezeichnung: Dicocoylethyl Hydroxyethylmonium Methosulfate), Dehyquart® F-75, Dehyquart® C-4046, und Dehyquart® AU-35 sind Beispiele für solche Esterquats. Zur Herstellung können auch Fettsäureschnitte, z.B. aus Talg wie beispielsweise Rindertalg gewonnene Talgfettsäuren, verwendet werden (INCI-Bezeichnungen: Ditallowoylethyl Dimonium Methosulfate, Ditallowoyl PG-dimonium Chloride).
- Salzen des N,N-Dimethyl-N-(2-hydroxyethyl)-N-(2-hydroxyhexadecyl)-ammoniums. Bevorzugte Halogenide sind Chlorid und Bromid. Diese werden beispielsweise unter der Handelsmarke Dehyquart® E (INCI-Bezeichnung: Aqua (Water), Hydroxycetyl Hydroxyethyl Dimonium Chloride) von der Firma Cognis vertrieben.
- quaternierte Amidsalze von Fettsäuren mit Diaminen, wie z.B. Salzen des N-(13-Docosen)amidopropyl-N-2-hydroxyethyl-N,N-dimethylammoniums (INCI-Bezeichnung: Hydroxyethyl Erucamidopropyl Dimonium Chloride) oder des N-Docosylamidopropyl-N-2-hydroxyethyl-N,N-dimethylammoniums welches unter dem Handelsnamen Incroquat® Behenyl HE (INCI-Bezeichnung: Hydroxyethyl Behenamidopropyl Dimonium Chloride) von der Firma Croda Inc. vertrieben wird.

Als quartäre Ammoniumverbindungen aus der Gruppe der Polymere, die sich ableiten von mindestens einem Monomer mit einer quartären Ammoniumgruppe, sind vorzugsweise kationische und/oder amphotere Polymere einsetzbar. Unter kationischen bzw. amphoteren Polymeren sind Polymere zu verstehen, welche in der Haupt- und/oder Seitenkette eine Gruppe aufweisen, die "permanent" kationisch ist. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine ($C_1$ bis $C_4$)-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (Q2),

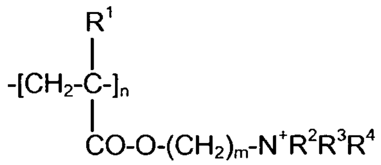

$X^-$ (Q2)

in der $R^1$= -H oder $-CH_3$ ist, $R^2$, $R^3$ und $R^4$ unabhängig voneinander ausgewählt sind aus ($C_1$ bis $C_4$)-Alkyl-, -Alkenyl- oder -Hydroxyalkylgruppen, m = 1, 2, 3 oder 4, n eine natürliche Zahl und $X^-$ ein physiologisch verträgliches organisches oder anorganisches Anion ist, sowie Copolymere, bestehend im wesentlichen aus den in Formel (Q2) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:

- $R^1$ steht für eine Methylgruppe
- $R^2$, $R^3$ und $R^4$ stehen für Methylgruppen
- m hat den Wert 2.

Als physiologisch verträgliches Gegenionen $X^-$ kommen beispielsweise Halogenidionen, Sulfationen, Phosphationen, Methosutfationen sowie organische lonen wie Lactat-, Citrat-, Tartrat- und Acetationen in Betracht. Bevorzugt sind Halogenidionen, insbesondere Chlorid.

Ein besonders geeignetes Homopolymer (Q2) ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare® CTH (Cosmetic Rheologies) und Synthalen® CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (Q2) enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Als erfindungsgemäße quartäre Ammoniumverbindung können die erfindungsgemäßen Mittel mindestens ein Polymerisat enthalten, das mindestens eine Struktureinheit gemäß Formel (Q3) umfasst,

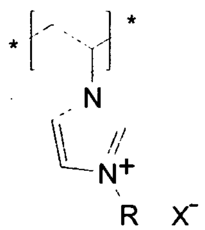
(Q3)

worin

R eine $C_1$- bis $C_{30}$-Alkylgruppe, eine $C_1$- bis $C_4$-Aralkylgruppe, eine $C_2$- bis $C_6$-Alkenylgruppe oder eine $C_2$ bis $C_6$-Hydroxyalkylgruppe bedeutet und
$X^-$ für ein physiologisch verträgliches Anion steht.

Als erfindungsgemäß bevorzugt geeignete Polymerisate der Formel (Q3) dienen solche, in der R für eine $C_1$- bis $C_{10}$-Alkylgruppe, insbesondere für eine Methylgruppe, steht.

Beispiele für erfindungsgemäß in Verbindungen der Formel (Q3) verwendbare $C_1$- bis $C_{30}$-Alkylgruppen sind Methyl, Ethyl, n-Propyl, Isopropyl, n-Butyl, sec-Butyl, Isobutyl, tert-Butyl, n-Decyl, n-Dodecyl, Stearyl oder Behenyl. Beispiele für erfindungsgemäß in Verbindungen der Formel (Q3) verwendbare $C_2$- bis $C_6$-Alkenylgruppen sind Vinyl, 2-Propen-1-yl (Allyl) oder 3-Buten-1-yl.
Beispiele für eine $C_1$- bis $C_4$-Aralkylgruppe sind die Benzylgruppe und die 2-Phenylethylgruppe. Beispiele für eine $C_2$ bis $C_6$-Hydroxyalkylgruppe sind die 2-Hydroxyethylgruppe und die 3-Hydroxypropylgruppe.

Die physiologisch verträglichen Anionen X- gemäß Formel (Q3) dürfen definitionsgemäß nicht nur eine negative Ladung tragen, sondern können auch eine Ladungszahl größer 1 besitzen. In letzterem Falle werden die Anionen $X^-$ der Salzform gemäß Formel (Q3) zur Wahrung der Elektroneutralität durch Formulierung eines stöchiometrischen Koeffizienten kleiner 1 vor dem Anion beschrieben. Die physiologisch verträglichen Anionen werden bevorzugt ausgewählt aus Halogenid, Benzolsulfonat, p-Toluolsulfonat, $C_1$-$C_4$-Alkansulfonat, Trifluormethansulfonat, $C_1$-$C_4$-Alkylsulfat, Perchlorat, 0.5 Sulfat, Hydrogensulfat, 0.5 Carbonat, Hydrogencarbonat, 1/3 Phosphat, 0.5 Hydrogenphosphat, Dihydrogenphosphat, Lactat, Citrat, Tetrafluoroborat, Hexafluorophosphat oder Tetrachlorozinkat. Besonders bevorzugt steht $X^-$ für ein Halogenid, insbesondere Chlorid, Bromid oder Iodid, $C_1$-$C_4$-Alkylsulfat, p-Toluolsulfonat, Tetrafluoroborat, Trifluormethansulfonat, Hexafluorophosphat, 0.5 Sulfat, oder Hydrogensulfat. Besonders bevorzugt werden die Anionen Chlorid, Methylsulfat oder Ethylsulfat als X- eingesetzt.

Die erfindungsgemäß verwendbaren Polymere der Formel (Q3) besitzen ein bevorzugtes Molekulargewicht von mehr als 100000 g/mol, besonders bevorzugt von mehr als 400000 g/mol.

Es ist erfindungsgemäß bevorzugt, ein Copolymerisat zu verwenden, das zusätzlich zu mindestens einer Struktureinheit gemäß obiger Formel (Q3) mindestens eine Struktureinheit der Formel (N1) umfasst,

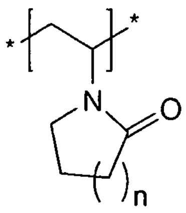

(N1)

worin n für 1, 2 oder 3 als Anzahl der Methyleneinheiten steht.

Bevorzugte Polymerisate sind unter den Handelsnamen Luviquat® FC 370, Luviquat® FC 550, Luviquat® FC 905 und Luviquat® HM 552, sowie Luviquat® Hold (INCI-Bezeichung: Polyquaternium-46), Luviquat® Supreme (Polyquaternium-68) und/oder Luviquat® Ultracare jeweils von der Firma BASF zu beziehen.

Weiterhin bevorzugt verwendbar ist ein Copolymer, in welchem neben mindestens einer Struktureinheit der Formel (Q3), mindestens eine Struktureinheit der Formel (N1) mit n = 1 und mindestens eine weitere Struktureinheit der Formel (N1) mit n = 3 enthalten ist.

Bevorzugt eignet sich ebenfalls ein Terpolymer mit den Struktureinheiten der Formeln,

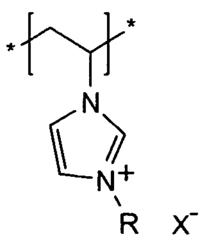

(Q3)

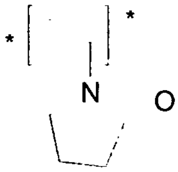

(N1-a)

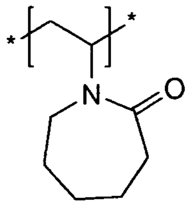

(N1-b)

in denen R und $X^-$ die für Formel (Q3) angegebenen Bedeutungen, insbesondere aller zuvor genannten Ausführungsformen, haben.

Solche Polymere werden beispielsweise von der Firma BASF unter dem Handelsnamen Luviquat® Hold (INCI-Bezeichnung: Polyquaternium-46) vertrieben. Die Gewichtsverhältnisse (in Gewichtsprozent) der Struktureinheiten der Formel (I) zu den Struktureinheiten der Formel (II) mit n = 1 zu den Struktureinheiten der Formel (II) mit n = 3 beträgt in dem Handelsprodukt 10 zu 40 zu 50 und die Ladungsdichte beträgt 0.5 meq/g.

In allen erfindungsgemäß verwendbaren Polymeren können bei Vorhandensein verschiedener Struktureinheiten diese jeweils statistisch verteilt oder als Block vorliegen.

Weitere in den erfindungsgemäß Mitteln einsetzbare kationische Polymere sind beispielsweise

- quaternisierte Cellulose-Derivate, wie sie unter den Bezeichnungen Celquat® und Polymer JR im Handel erhältlich sind. Die Verbindungen Celquat H 100, Celquat L 200 und Polymer JR 400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Zusätzlich zu kationischen Polymerisaten oder an ihrer Stelle können die erfindungsgemäßen Mittel auch amphotere Polymere enthalten. Diese weisen zusätzlich mindestens eine negativ geladene Gruppe im Molekül auf und werden auch als zwitterionische Polymere bezeichnet. Im Rahmen der vorliegenden Erfindung bevorzugt einsetzbare zwitterionische Polymerisate (Q4) setzen sich im wesentlichen zusammen aus

A) Monomeren mit quartären Ammoniumgruppen der allgemeinen Formel (Z1-a),

$$R^1\text{-CH=CR}^2\text{-CO-Z-}(C_nH_{2n})\text{-N}^{(+)}R^3R^4R^5\ A^{(-)} \qquad (Z1\text{-a})$$

In der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Köhlenstoff-Atomen, Z eine NH-Gruppe oder ein Sauerstoffatom, n eine ganze Zahl von 2 bis 5 und $A^{(-)}$ das Anion einer organischen oder anorganischen Säure ist
und

B) monomeren Carbonsäuren der allgemeinen Formel (Z1-b),

$$R^6\text{-CH=CR}^7\text{-COOH} \qquad (Z1\text{-b})$$

in denen $R^6$ und $R^7$ unabhängig voneinander Wasserstoff oder
Methylgruppen sind.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z gemäß Formel (Z1-a) eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert, wobei als Alkylierungsreagenzien Methylchlorid, Dimethylsulfat oder Diethylsulfat besonders geeignet sind. Die Quaternisierungsreaktion kann in wäßriger Lösung oder im Lösungsmittel erfolgen.

Vorteilhafterweise werden solche Monomere der Formel (Z1-a), die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (Z1-a), bei denen $R^3$, $R^4$ und $R^5$ Methylgruppen sind.

Das Acrylamidopropyl-trimethylammoniumchlorid ist ein ganz besonders bevorzugtes Monomer der Formel (Z1-a).

Als monomere Carbonsäuren der Formel (Z1-b) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die erfindungsgemäß einsetzbaren zwitterionischen Polymerisate werden aus Monomeren der Formeln (Z1-a) und (Z1-b) nach an sich bekannten Polymerisationsverfahren hergestellt. Die Polymerisation kann entweder in wäßriger oder wäßrig-alkoholischer Lösung erfolgen. Als Alkohole werden Alkohole mit 1 bis 4 Kohlenstoffatomen, vorzugsweise Isopropanol, verwendet, die gleichzeitig als Polymerisationsregler dienen. Der Monomerlösung können aber auch andere Komponenten als Regler zugesetzt werden, z. B. Ameisensäure oder Mercaptane, wie Thioethanol und Thioglykolsäure. Die Initiierung der Polymerisation erfolgt mit Hilfe von radikalbildenden Substanzen. Hierzu können Redoxsysteme und/oder thermisch zerfallende Radikalbildner vom Typ der Azoverbindungen, wie z. B. Azoisobuttersäurenitril, Azo-bis-(cyanopentansäure) oder Azo-bis-(amidinopropan)dihydrochlorid verwendet werden. Als Redoxsysteme eignen sich z. B. Kombinationen aus Wasserstoffperoxid, Kalium- oder Ammoniumperoxodisulfat sowie tertiäres Butylhydroperoxid mit Natriumsulfit, Natriumdithionit oder Hydroxylaminhydrochlorid als Reduktionskomponente.

Die Polymerisation kann isotherm oder unter adiabatischen Bedingungen durchgeführt werden, wobei in Abhängigkeit von den Konzentrationsverhältnissen durch die freiwerdende Polymerisationswärme der Temperaturbereich für den Ablauf der Reaktion zwischen 20 und 200 °C schwanken kann, und die Reaktion gegebenenfalls unter dem sich einstellenden Überdruck durchgeführt werden muß. Bevorzugterweise liegt die Reaktionstemperatur zwischen 20 und 100 °C.

Als besonders wirksam haben sich erfindungsgemäß solche Polymerisate erwiesen, bei denen die Monomeren der Formel (Z1-a) gegenüber den Monomeren der Formel (Z1-b) im Überschuß vorlagen. Es ist daher erfindungsgemäß bevorzugt, solche Polymerisate zu verwenden, die aus Monomeren der Formel (Z1-a) und die Monomeren der Formel

(Z1-b) in einem Molverhältnis von 60:40 bis 95:5, insbesondere von 75:25 bis 95:5, bestehen.

Aus der Gruppe der organischen N-Oxide erfindungsgemäß besonders geeignete Verbindung sind Verbindungen mit der Formel (Q5),

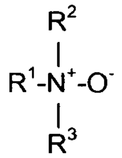

(Q5)

worin bedeuten

$R^1$ und $R^2$ unabhängig voneinander eine lineare oder verzweigte ($C_1$ bis $C_{30}$)-Alkylgrupppe, eine Aryl-($C_1$ bis $C_6$)-alkylgruppe, eine ($C_2$ bis $C_{30}$)-Acylgruppe, oder eine Gruppe - $A^1$-$A^2$-$A^3$-$R^5$
worin
$R^5$ eine lineare oder verzweigte ($C_8$ bis $C_{30}$)-Alkylgruppe oder eine lineare oder verzweigte ($C_8$ bis $C_{30}$)-Alkenylgruppe bedeutet,
$A^1$ eine direkte Bindung oder eine Carbonylgruppe bedeutet,
$A^2$ eine direkte Bindung, eine Gruppe NH oder ein Sauerstoffatom bedeutet,
$A^3$ eine ($C_2$ bis $C_4$)-Alkylengruppe oder eine ($C_2$ bis $C_4$)-Hydroxyalkylengruppe bedeutet und

$R^3$ eine lineare oder verzweigte ($C_8$ bis $C_{30}$)-Alkylgruppe, eine ($C_8$ bis $C_{30}$)-Hydroxyalkylgruppe, eine ($C_2$ bis $C_{30}$)-Acylgruppe,

wobei $R^1$ und $R^2$ auch gemeinsam mit dem Restmolekül einen fünf- oder sechsgliedrigen Ring bilden können.

Bevorzugt einsetzbare Aminoxide sind Dimethyl($C_8$-bis $C_{22}$)alkylaminoxide. Insbesondere werden Cocamidopropylaminoxid, N-Cocomorpholinoxid, Decyldimethylaminoxid, Dimethylcetylaminoxid, Dimethylcocoaminoxid (Aromox® MCDW, Fa. Akzo Nobel), Dimethylhydriertes-Talgaminoxid, Dimethyllaurylaminoxid, Dimethylmyristylaminoxid, (2-Hydroxyethyl)cocoaminoxid oder Oleaminoxid bevorzugt in den erfindungsgemäßen Mitteln als nichtionische N-Oxid-Tenside eingesetzt.

Weiterhin können erfindungsgemäß als quartäre Ammoniumverbindungen aus der Gruppe der organischen N-Oxide mindestens ein Pyridin-N-oxid oder Derivate davon, ein Pyrimidin-N-Oxid oder Derivate davon, wie insbesondere 2,4-Diaminopyrimidine-3-oxid (Fa. Chimex), in dem erfindungsgemäßen Mittel enthalten sein.

Zusätzlich zu der erfindungsgemäßen Wirkstoffkombination können die erfindungsgemäßen Mittel als weitere Komponente mindestens eine Verbindung aus den Aldehyden und/oder den Ketonen enthalten.

Der Auswahl der Verbindungen aus den Aldehyden bzw. den Ketonen ist zunächst keine Grenze gesetzt. Als besonders geeignet erweist sich mindestens ein Vertreter aus der Gruppe:

- Oxocarbonsäuren oder deren Salze,
- Oxocarbonsäureester,
- cyclische, lineare oder verzweigte aliphatische Aldehyde,
- cyclische, lineare oder verzweigte aliphatische Ketone,
- mono- bis polyhydroxyfunktionalisierte Aldehyde,
- mono- bis polyhydroxyfunktionalisierte Ketone,
- alicyclische, aromatische oder heterocyclische Aldehyde sowie
- alicyclische, aromatische oder heterocyclische Ketone.

Generell gilt, dass in den oben genannten Verbindungen der cyclischen, alicyclischen bzw. heterocyclischen Ketone die Ketogruppe(n) endocyclisch (und/oder) oder exocyclisch gebunden sein kann (können).

Oxocarbonsäuren sind organische Verbindungen, die neben mindestens einer Carboxylgruppe eine Carbonylgruppe tragen und sind somit Aldehyd- bzw. Ketocarbonsäuren. Bevorzugte Oxocarbonsäuren sind $\alpha$-Oxocarbonsäuren, $\beta$-Oxocarbonsäuren, $\gamma$-Oxocarbonsäuren sowie w-Oxocarbonsäuren der Formel (XII) bzw. deren Salze,

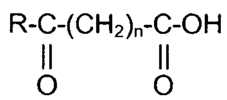

(XII)

worin bedeuten

R ein Wasserstoffatom, eine ($C_1$ bis $C_6$)-Alkylgruppe, eine ($C_1$ bis $C_6$)-Hydroxyalkylgruppe, eine gegebenenfalls substituierte Arylgruppe, eine gegebenenfalls substituierte Heteroarylgruppe, eine ($C_2$ bis $C_6$)-Alkenylgruppe oder eine Carboxy-($C_1$ bis $C_6$)-alkylgruppe,

n eine Zahl 0, 1, 2 oder 3.

Besonders bevorzugt werden die Oxocarbonsäuren ausgewählt aus mindestens einem Vertreter aus der Gruppe Glyoxalsäure, Acetessigsäure, 3-Oxoglutarsäure, 4-Oxovaleriansäure und Brenztraubensäure bzw. den Salzen der vorgenannten Säuren.

Die Oxocarbonsäureester werden bevorzugt ausgewählt aus ($C_1$ bis $C_6$)-Alkylestern der erfindungsgemäß bevorzugten Oxocarbonsäuren.

Unter cyclischen, linearen oder verzweigten aliphatischen Aldehyden sind erfindungsgemäß aliphatische Aldehyde zu verstehen, deren Formylgruppe(n) nicht in Konjugation mit einem aromatischen $\pi$-Elektronen-System stehen. Die entsprechenden Aldehyde dürfen aromatische Reste tragen, solange die $\pi$-Elektronen der Formylgruppe(n) nicht über ein solches aromatisches System delokalisiert sein können. Bevorzugte cyclische, lineare oder verzweigte aliphatische Aldehyde sind gesättigt oder ungesättigt und werden besonders bevorzugt ausgewählt aus mindestens einem Vertreter aus der Gruppe Formaldehyd, Acetaldehyd, Glyoxal, Propionaldehyd, Butanal, Pentanal, Isopentanal, Hexanal, Cyclohexanal, Heptanal, Octanal, Malondialdehyd, Glutaraldehyd, 2-Methylpentanal, 2-Ethylhexanal, 3,5,5-Trimethylhexanal, 2-Ethylbutyraldehyd, 2-Methylbutyraldehyd, Isobutyraldehyd, 3-Phenylpropanal, 3-(4-Methylphenyl)propanal, 3-(4-Methoxyphenyl)propanal, 3-(2-Methoxyphenyl)propanal, 2-Butenal, Acrolein, 3-Methyl-2-butenal, 3,7-Dimethyl-2,6-octadienal, 2,4-Pentadienal, 3,7-Dimethyl-6-octenal, 2,4-Dimethyl-3-cyclohexencarboxaldehyd und 2,6-Nonadienal.

Unter cyclischen, linearen oder verzweigten aliphatischen Ketonen sind erfindungsgemäß aliphatische Ketone zu verstehen, deren Ketogruppe(n) nicht in Konjugation mit einem aromatischen $\pi$-Elektronen-System stehen. Die entsprechenden Ketone dürfen jedoch aromatische Reste tragen, solange die $\pi$-Elektronen der Ketogruppe(n) nicht über ein solches aromatisches System delokalisiert sein können. Bevorzugte cyclische, lineare oder verzweigte aliphatische Ketone sind gesättigt oder ungesättigt und ausgewählt aus mindestens einem Vertreter aus der Gruppe Aceton, 2-Butanon, 2-Pentanon, 3-Pentanon, 2-Hexanon, 3-Hexanon, Butan-2,4-dion, Pentan-2,4-dion, Hexan-2,5-dion, Cyclohexanon, Cyclopentanon, 4-Methylpentan-2-on, 5-Methyl-3-hexen-2-on, 2-(3-Oxopropyl)benzoesäuremethylester und 4-(3-Oxopropyl)benzoesäuremethylester.

Bevorzugt verwendbare monohydroxyfunktionalisierte Aldehyde werden ausgewählt aus mindestens einem Vertreter aus der Gruppe 1-Hydroxypropanal, 5-Hydroxypentanal, 3,7-Dimethyl-7-hydroxyoctanal, Hydroxyisohexyl-3-cyclohexen-1-carboxaldehyd und 2,6,6-Trimethyl-1,3-cyclohexadien-1-carboxaldehyd.

Polyhydroxyfunktionalisierte Aldehyde sind erfindungsgemäß bevorzugt die sogenannten Aldosen und werden besonders bevorzugt ausgewählt aus mindestens einem Vertreter aus der Gruppe 2,3-Dihydroxypropionaldehyd, D-Erythrose, D-Threose, D-Ribose, D-Arabinose, D-Lyxose, D-Xylose, D-Allose, D-Altrose, D-Galactose, D-Glucose, D-Idose, D-Mannose, D-Rhamnose und D-Talose, sowie den L-Konfigurationen L-Erythrose, L-Threose, L-Ribose, L-Arabinose, L-Lyxose, L-Xylose, L-Allose, L-Altrose, L-Galactose, L-Glucose, L-Idose, L-Mannose, L-Rhamnose und L-Talose.

Monohydroxyfunktionalisierte Ketone, die sich erfindungsgemäß besonders eignen, werden bevorzugt ausgewählt aus mindestens einem Vertreter aus der Gruppe 1-Hydroxy-2-propanon, 1-Hydroxy-2-butanon, 3-Hydroxy-2-butanon und Benzoin.

Polyhydroxyfunktionalisierte Ketone sind erfindungsgemäß bevorzugt die sogenannten Ketosen und werden besonders bevorzugt ausgewählt aus mindestens einem Vertreter aus der Gruppe 1,3-Dihydroxyaceton, D-Psicose, D-Fructose, D-Sorbose, D-Tagatose, D-Ribulose, D-Xylulose, D-Erythrulose sowie den L-Konfigurationen L-Psicose, L-Fructose, L-Sorbose, L-Tagatose, L-Ribulose, L-Xylulose, L-Erythrulose.

Bevorzugte alicyclische, aromatische oder heterocyclische Ketone werden bevorzugt ausgewählt aus mindestens einem Vertreter aus der Gruppe

- Benzylidenketone, insbesondere den Benzylidenketonen aus der Patentanmeldung DE-A1-19717281 (siehe DE-A1-19717281: Formel (I) des 1. Anspruchs und die Vertreter gemäß 4. Anspruch), auf die ausdrücklich Bezug genommen wird,
- Imidazolin-2,4-dion und dessen Derivaten, insbesondere Allantoin und/oder 5,5-Dimethylhydantoin.

Bevorzugte alicyclische, aromatische oder heterocyclische Aldehyde werden bevorzugt ausgewählt aus mindestens einem Vertreter aus der Gruppe

- Benzaldehyd und seinen Derivaten,

- Zimtaldehyd und seinen Derivaten sowie
- Naphthaldehyd und seinen Derivaten.

Die Derivate der Benzaldehyde, Naphthaldehyde bzw. Zimtaldehyde werden bevorzugt ausgewählt aus mindestens einem Vertreter aus der Gruppe Benzaldehyd, 1-Naphthaldehyd, Coniferylaldehyd, 2-Methylbenzaldehyd, 3-Methylbenzaldehyd, 4-Methylbenzaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methylbenzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 3-Hydroxy-2-methoxy-benzaldehyd, 2-Hydroxy-4-methoxy-benzaldehyd, 2-Ethoxy-4-hydroxy-benzaldehyd, 3-Ethoxy-4-hydroxybenzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxybenzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-düodbenzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-(1-Imidazolyl)-benzaldehyd und Piperonal.

Alle Aldehyd bzw. Ketoverbindungen sollen aus physiologisch verträglichen bzw. nicht toxischen Verbindungen ausgewählt werden, wenn das erfindungsgemäße Mittel als kosmetisches Mittel Verwendung finden soll.

Die Verbindungen, ausgewählt aus Aldehyden und/oder Ketonen, sind erfindungsgemäß bevorzugt in einer Menge von 0,1 Gew.-% bis 20 Gew.-%, insbesondere von 0,5 Gew.-% bis 10 Gew.-%, jeweils bezogen auf das Gewicht des anwendungsbereiten Mittels, in dem erfindungsgemäßen Mittel enthalten.

Es kann erfindungsgemäß bevorzugt sein, das erfindungsgemäße Mittel mit der Maßgabe bereitzustellen, dass die in dem erfindungsgemäßen Mittel eingesetzten Verbindungen, ausgewählt aus den Aldehyden und/oder den Ketonen, sich von denjenigen Aldehyden bzw. Ketonen unterscheiden müssen, von denen sich diejenigen Sulfinsäuren ableiten, die in dem erfindungsgemäßen Mittel tatsächlich enthalten sind.

Es ist ebenso erfindungsgemäß bevorzugt, wenn das erfindungsgemäße Mittel zusätzlich mindestens ein Redukton enthält. Unter einem Redukton versteht der Fachmann reduktiv wirkende Endiol-Verbindungen, die durch Substitution in $\alpha$-Stellung stabilisiert sind und die der Tautomerie unterliegen. Die wichtigsten erfindungsgemäß einsetzbaren Reduktone sind Ascorbinsäure, Isoascorbinsäure, 2,3-Dihydroxy-2-propendial und 2,3-Dihydroxy-2-cyclopentenon.

Die Reduktone sind erfindungsgemäß bevorzugt in einer Menge von 0,1 Gew.-% bis 20 Gew.-%, insbesondere von 0,5 Gew.-% bis 10 Gew.-%, jeweils bezogen auf das Gewicht des anwendungsbereiten Mittels, in dem erfindungsgemäßen Mittel enthalten.

Als kosmetische Träger für das erfindungsgemäße Mittel eignen sich bevorzugt flüssige Medien, in denen das erfindungsgemäße Sulfinsäurederivat bevorzugt löslich ist, wie beispielsweise Wasser oder organische Lösemittel.

Als kosmetische Träger eignen sich besonders Cremes, Emulsionen, Gele oder auch tensidhaltige schäumende

Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägem sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen. Weitere alkoholische Lösemittel, sind beispielsweise Methoxybutanol, Benzylalkohol, 2-Phenoxyethanol, Ethyldiglykol oder 1,2-Propylenglykol. In einer bevorzugten Ausführungsform enthält das erfindungsgemäße Mittel zusätzlich als Lösemittel mindestens einen ($C_2$ bis $C_6$)-Alkylmonoalkohol und/oder ein ($C_2$ bis $C_6$)-Alkandiol, insbesondere Ethanol, Isopropanol und/oder 1,2-Propylenglykol.

Das erfindungsgemäße Mittel besitzt bevorzugt einen pH-Wert von pH 1 bis pH 9, insbesondere von pH 1,5 bis pH 6.

Ein zweiter Gegenstand der Erfindung ist die Verwendung des Mittels gemäß erstem Erfindungsgegenstand zur Entfärbung von Haut und/oder keratinhaltigen Fasern, insbesondere menschlichem Haar, die jeweils mit natürlichen und/oder synthetischen Farbstoffen eingefärbt sind.

Unter keratinhaltigen Fasern werden erfindungsgemäß insbesondere Wolle oder tierische oder menschliche Haare, ganz besonders bevorzugt menschliche Haare, verstanden.

Die zu entfärbenden Substrate sind bevorzugt mit Oxidationsfarbstoffen und/oder direktziehenden Farbstoffen, als Vertreter der synthetischen Farbstoffe, gefärbt.

Als zur Färbung des Substrats verwendete Entwicklerkomponenten können prinzipiell nachfolgende Entwicklerkomponenten dienen.

Besonders bevorzugt sind p-Phenylendiaminderivate der Formel (E1)

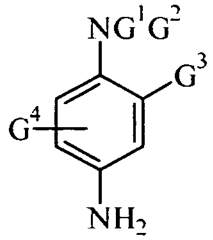

(E1)

wobei

- $G^1$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen 4'-Aminophenylrest oder einen $C_1$- bis $C_4$-Alkylrest, der mit einer stickstoffhaltigen Gruppe, einem Phenyl- oder einem 4'-Aminophenylrest substituiert ist;
- $G^2$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest oder einen $C_1$- bis $C_4$-Alkylrest, der mit einer stickstoffhaltigen Gruppe substituiert ist;
- $G^3$ steht für ein Wasserstoffatom, ein Halogenatom, wie ein Chlor-, Brom-, Iod- oder Fluoratom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Hydroxyalkoxyrest, einen $C_1$- bis $C_4$-Acetylaminoalkoxyrest, einen $C_1$- bis $C_4$- Mesylaminoalkoxyrest oder einen $C_1$- bis $C_4$-Carbamoylaminoalkoxyrest;
- $G^4$ steht für ein Wasserstoffatom, ein Halogenatom oder einen $C_1$- bis $C_4$-Alkylrest oder
- wenn $G^3$ und $G^4$ in ortho-Stellung zueinander stehen, können sie gemeinsam eine verbrückende $\alpha,\omega$-Alkylendioxogruppe, wie beispielsweise eine Ethylendioxygruppe bilden.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N, N-Dipropyl-p-phenylendiamin, 4-Amino-3-methyl-(N,N-diethyl)-anilin, N,N-Bis-(β-hydroxyethyl)-p-phenylendiamin, 4-N, N-Bis-(β-hydroxyethylramino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N, N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethytoxy)-p-phenylendiamin, 2-(β-Acetylaminoethyloxy)-p-phenylendiamin, N-(β-Methoxyethyl)-p-phenylendiamin, N-(4-Amino-3-methylphenylrN-[3-(1H-imidazol-1-yl)propyl]amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin zur Färbung des Substrats bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze:

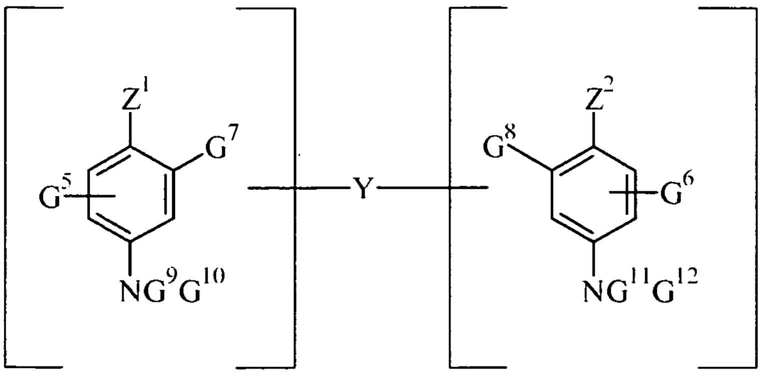

(E2)

wobei:

- $Z^1$ und $Z^2$ stehen unabhängig voneinander für einen Hydroxyl- oder $NH_2$-Rest, der gegebenenfalls durch einen $C_1$- bis $C_4$-Alkylrest, durch einen $C_1$- bis $C_4$-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder $C_1$- bis $C_8$-Alkoxyreste substituiert sein kann, oder eine direkte Bindung,
- $G^5$ und $G^6$ stehen unabhängig voneinander für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Aminoalkylrest oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder einen $C_1$- bis $C_4$-Alkylrest,

mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze zur Färbung des zu entfärbenden Substarts einzusetzen. Besonders

bevorzugt sind p-Aminophenolderivate der Formel (E3)

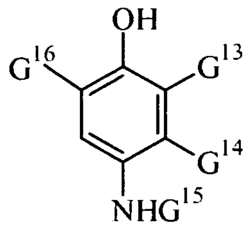

(E3)

wobei:

- $G^{13}$ steht für ein Wasserstoffatom, ein Halogenatom, einen $C_1$- bis $C_4$-Alkylrest, einen $C_1$-bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest, einen Hydroxy-($C_1$- bis $C_4$)-alkylaminorest, einen $C_1$- bis $C_4$-Hydroxyalkoxyrest, einen $C_1$- bis $C_4$-Hydroxyalkyl-($C_1$-bis $C_4$)-aminoalkylrest oder einen (Di-$C_1$- bis $C_4$-Alkylamino)-($C_1$- bis $C_4$)-alkylrest, und
- $G^{14}$ steht für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$-bis $C_4$)-alkylrest, einen $C_1$- bis $C_4$-Aminoalkylrest oder einen $C_1$- bis $C_4$-Cyanoalkylrest,
- $G^{15}$ steht für Wasserstoff, einen $C_1$- bis $C_4$-Alkylrest, einen $C_1$- bis $C_4$-Monohydroxyalkylrest, einen $C_2$- bis $C_4$-Polyhydroxyalkylrest, einen Phenylrest oder einen Benzylrest, und
- $G^{16}$ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethylrpyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazolopyrimidin-Derivate sind insbesondere die Derivate des Pyrazolo[1,5-a]pyrimidin der folgenden Formel (E4) und dessen tautomeren Formen, sofern ein tautomeres Gleichgewicht besteht:

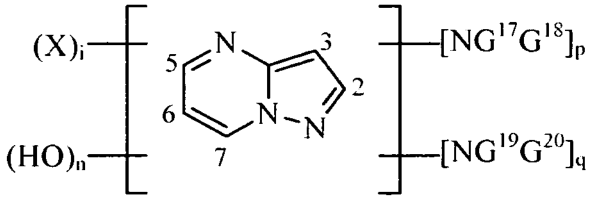

(E4)

wobei:

- $G^{17}$, $G^{18}$, $G^{19}$ und $G^{20}$ unabhängig voneinander stehen für ein Wasserstoffatom, einen $C_1$-bis $C_4$-Alkylrest, einen Aryl-Rest, einen $C_1$- bis $C_4$-Hydroxyalkylrest, einen $C_2$- bis $C_4$-Polyhydroxyalkylrest einen ($C_1$- bis $C_4$)-Alkoxy-($C_1$- bis $C_4$)-alkylrest, einen $C_1$- bis $C_4$-Aminoalkylrest, der gegebenenfalls durch ein Acetyl-Ureid- oder einen Sulfonyl-Rest geschützt sein kann, einen ($C_1$- bis $C_4$)-Alkylamino-($C_1$- bis $C_4$)-alkylrest, einen Di-[($C_1$- bis $C_4$)-alkyl]-($C_1$-bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$- bis $C_4$)-[Hydroxyalkyl]-($C_1$- bis $C_4$) aminoalkylrest,
- die X-Reste stehen unabhängig voneinander für ein Wasserstoffatom, einen $C_1$- bis $C_4$-Alkylrest, einen Aryl-Rest, einen $C_1$- bis $C_4$-Hydroxyalkylrest, einen $C_2$- bis $C_4$-Polyhydroxyalkylrest, einen $C_1$- bis $C_4$-Aminoalkylrest, einen ($C_1$- bis $C_4$)-Alkylamino-($C_1$- bis $C_4$)-alkylrest, einen Di-[($C_1$- bis $C_4$)alkyl]- ($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$- bis $C_4$-hydroxyalkyl)-aminoalkylrest, einen Aminorest, einen $C_1$- bis $C_4$-Alkyl- oder Di-($C_1$- bis $C_4$-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,

mit der Maßgabe, dass

- die Summe aus p + q ungleich 0 ist,
- wenn p + q gleich 2 ist, n den Wert 0 hat, und die Gruppen $NG^{17}G^{18}$ und $NG^{19}G^{20}$ belegen die Positionen (2,3); (5,6); (6,7); (3,5) oder (3,7);
- wenn p + q gleich 1 ist, n den Wert 1 hat, und die Gruppen $NG^{17}G^{18}$ (oder $NG^{19}G^{20}$) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazolo[1,5-a]pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

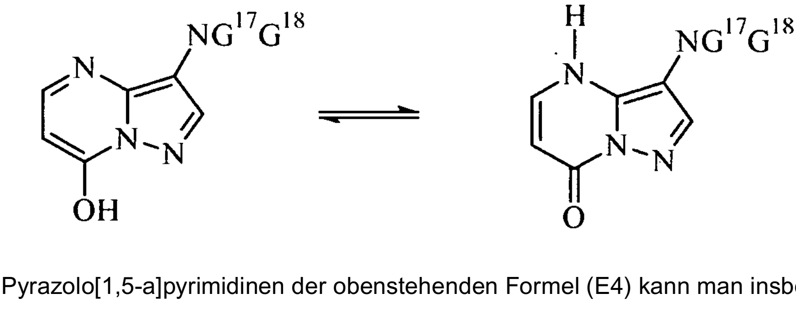

Unter den Pyrazolo[1,5-a]pyrimidinen der obenstehenden Formel (E4) kann man insbesondere nennen:

- Pyrazolo[1,5-a]pyrimidin-3,7-diamin;
- 2,5-Dimethylpyrazolo[1,5-a]-pyrimidin-3,7-diamin;

- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;

sowie ihre physiologisch verträglichen Salze und ihre tautomeren Formen, wenn ein tautomeres Gleichgewicht vorhanden ist.

Die Pyrazolo[1,5-a]pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform wurden die zu entfärbenden Substrate mit einem oxidativen Färbemittel gefärbt, welches neben mindestens einer Entwicklerkomponente zusätzlich mindestens eine Kupplerkomponente enthält.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind

- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethylramino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenylrpropan, 2,6-Bis-(2'-hydroxyethylamino)-1-methylbenzol, 2-({3-[(2-Hydroxyethyl)amino]-4-methoxy-5-methylphenyl}amino) ethanol, 2-({3-[(2-Hydroxyethyl)amino]-2-methoxy-5-methylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol

sowie deren physiologisch verträglichen Salze.

Erfindungsgemäß besonders bevorzugte Kupplerkomponenten sind ausgewählt aus mindestens einem Vertreter der Gruppe, die gebildet wird aus 1-Naphthol, 1,5-Dihydroxynaphthalin, 2,7-Dihydroxynaphthalin, 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Das zu entfärbende Substrat kann ebenso mit direktziehenden Farbstoffen eingefärbt worden sein. Als direktziehende Farbstoffe kommen dabei insbesondere Nitrophenylendiamine, Nitroaminophenole, Azofarbstoffe, Anthrachinone oder Indophenole in Frage. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die zu entfärbenden Substrate bevorzugt mit einem kationischen direktziehenden Farbstoff gefärbt sein. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

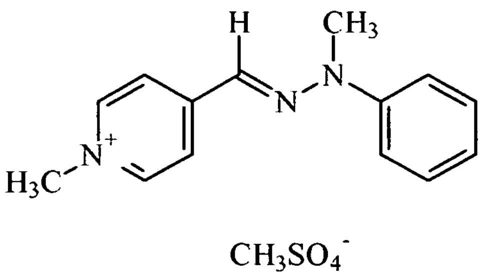

(DZ1) (Basic Yellow 87)

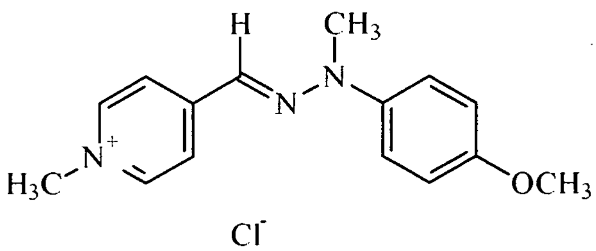

(DZ2)

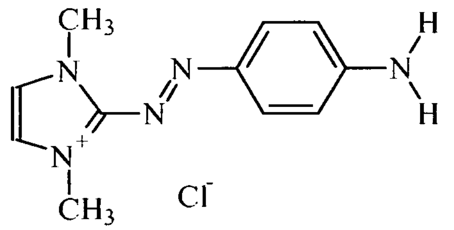

(DZ3) (Basic Orange 31)

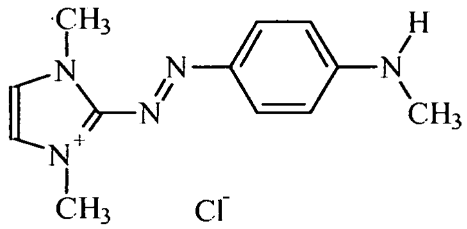

(DZ4)

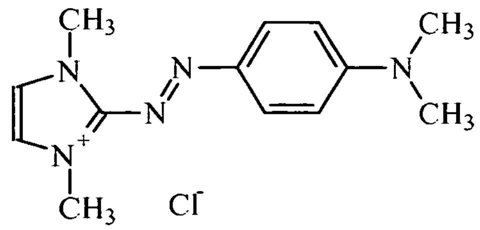

(DZ5) (Basic Red 51)

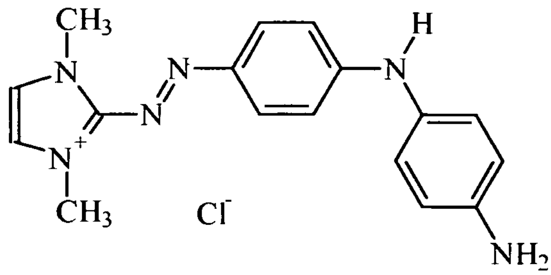

(DZ6)

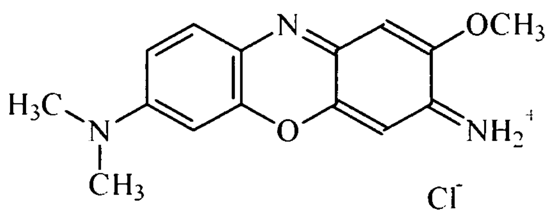

(DZ7)

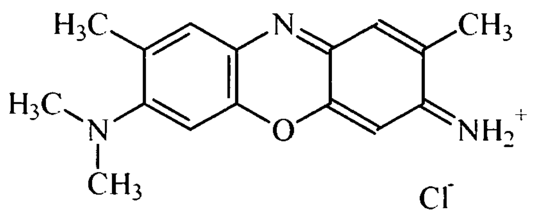

(DZ8)

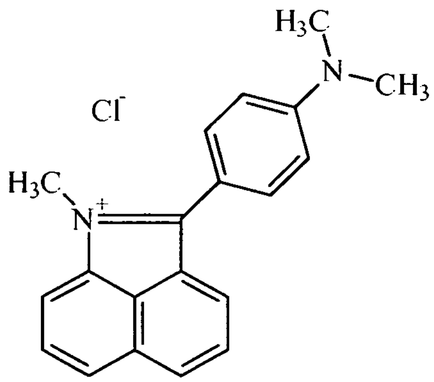

(DZ9)

Die Verbindungen der Formeln (DZ1), (DZ3) und (DZ5) sind ganz besonders bevorzugte kationische direktziehende Farbstoffe der Gruppe (c). Die kationischen direktziehenden Farbstoffe, die unter dem Warenzeichen Arianor® vertrieben werden, sind erfindungsgemäß besonders bevorzugte direktziehende Farbstoffe.

Weiterhin können die zu entfärbenden Substrate auch mit in der Natur vorkommenden, natürlichen Farbstoffen, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, gefärbt sein.

Ein zweiter Gegenstand der Erfindung ist ein Verfahren zur reduktiven Entfärbung von jeweils mit natürlichen und/oder synthetischen Farbstoffen eingefärbter Haut und/oder eingefärbtem Haar, in dem ein Mittel des ersten Erfindungsgegenstandes auf das eingefärbte Substrat aufgetragen wird und nach einer Einwirkzeit wieder abgespült wird. Als eingefärbtes Substrat wird jeweils mit natürlichen und/oder synthetischen Farbstoffen eingefärbte Haut und/oder eingefärbtes Haar definiert.

Die Einwirkzeit beträgt bevorzugt 1 bis 60 Minuten, bevorzugt 5 bis 30 Minuten. Die Einwirkung des erfindungsgemäßen Mittels kann nicht nur bei Raumtemperatur, sondern bevorzugt in einem Temperaturbereich von 15 bis 60 °C, insbesondere von 25 bis 60 °C erfolgen.

Nach Ablauf der Einwirkzeit werden die Haut bzw. die Haare ausgespült, wobei bevorzugt ein tensidhaltiges Mittel, wie beispielsweise ein Reinigungsmittel oder ein Shampoo, Anwendung findet. Gegebenenfalls kann die Haut bzw. das Haar mehrfach ausgespült, bzw. mehrfach mit dem tensidhaltigen Mittel behandelt werden.

Das tensidhaltige Mittel zur Spülung bzw. Nachbehandlung enthält bevorzugt in einem Träger mindestens ein Tensid, ausgewählt aus anionischen, zwitterionischen, ampholytischen, nichtionischen oder kationischen Tensiden, insbesondere aus nichtionischen Tensiden.

Als anionische Tenside eignen sich in den erfindungsgemäßen tensidhaltigen Mitteln zur Spülung bzw. Nachbehandlung alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R-O-(CH_2-CH_2O)_x-CH_2-COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R-O(CH_2-CH_2O)_x-SO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,

- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -$COO^{(-)}$- oder -$SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer $C_{8-18}$-Alkyl- oder -Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12-22}$-Fettsäuremono- und -diester von Anlagerungsprodukten von 1 bis 30 Mol Ethylenoxid an Glycerin,
- $C_{8-22}$-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen tensidhaltigen Mitteln zur Spülung bzw. Nachbehandlung verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methyl-hydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Die Tenside werden bevorzugt in Konzentrationen von 0,5 bis 30 Gew.-%, bezogen auf das Gewicht der Zusammensetzung, in dem tensidhaltigen Mittel zur Spülung bzw. Nachbehandlung eingesetzt.

Zusätzlich besitzt das tensidhaltige Mittel zur Spülung bzw. Nachbehandlung einen pH-Wert von kleiner pH 7. Dieser pH-Wert wird bevorzugt durch den Zusatz eines Puffers während des Spül- bzw. Nachbehandlungsschritts konstant gehalten. Erfindungsgemäß bevorzugt geeignete Puffersysteme sind der Phosphatpuffer ($PO_4^{3-}$ / $HPO_4^{2-}$ / $H_2PO_4^-$ / $H_3PO_4$), Essigsäure/AcetatPuffer, Citronensäure/Citrat-Puffer, KCl / HCl-Puffer, Monophthalat/HCl-Puffer oder Monophthalat/NaOH-Puffer.

Nach dem Ausspülen kann es vorteilhaft sein, mit einer Oxidationsmittelhaltigen Zusammensetzung nach zu behandeln. Bevorzugt wird Wasserstoffperoxid als Oxidationsmittel, bevorzugt in Konzentrationen von 0,5 bis 6 Gew.-%, eingesetzt. Die Einwirkzeit beträgt bevorzugt 1 bis 30 Minuten, besonders bevorzugt 1 bis 10 Minuten. Nach Ablauf der Einwirkzeit wird die Oxidationsmittelhaltige Zusammensetzug ausgespült.

Als bevorzugte natürliche oder synthetische Farbstoffe wurden zur Färbung der zu entfärbenden Haut und/ oder des zu entfärbenden Haars bevorzugt diejenigen Farbstoffe verwendet, wir sie im zweiten Erfindungsgegenstand definiert wurden.

Die Erfindung soll exemplarisch anhand der folgenden Ausführungen erläutert werden.

## Beispiele

### 1.1 **Ausfärbungen**

Für die Beurteilung der Entfärbung wurden Haarsträhnen von ca. 6 cm Länge und 0,5 g Gewicht des Codes Kerling Euronaturhaar weiß mit Farbstoffkombinationen aus

F1 p-Toluylendiamin / 5-Amino-2-methylphenol
F2 p-Toluylendiamin / 2,4-Diaminophenoxyethanol
F3 p-Toluylendiamin / Resorcin

gefärbt. Dazu wurden die Färbecremes F1 bis F3 nach bekanntem Herstellungsverfahren bereitgestellt, die jeweils die oben genannten Entwickler-Kuppler-Kombination enthalten:

Färbecreme:

| Rohstoff | Gew.% |
|---|---|
| Fettalkoholgemisch C12-C18 | 10,5 |
| Texapon NSO [1] | 20,0 |
| Dehyton K [2] | 12,5 |
| Eumulgin B2 [3] | 0,75 |
| Natriummetabisulfit | 1,0 |
| Ammoniumsulfat | 1,0 |
| Entwickler | 2,5 mMol |
| Kuppler | 2,5 mMol |

(fortgesetzt)

| Rohstoff | Gew.% |
|---|---|
| Wasser | ad 100 |
| [1] Laurylethersulfat, Natriumsalz (ca. 27,5% Aktivsubstanz; INCI-Bezeichnung: Sodium Laureth Sulfate) (Cognis)<br>[2] N,N-Dimethyl-N-(C8-18-kokosamidopropyl)ammoniumaceto-betain (ca. 30% Aktivsubstanz; INCI-Bezeichnung: Aqua (Water), Cocamidopropyl Betaine) (Cognis)<br>[3] Cetylstearylalkohol mit ca. 20 EO-Einheiten (INCI-Bezeichnung: Ceteareth-20) (Cognis) | |

Die Färbecreme wurde mit Ammoniaklösung (25%ig) auf einen pH-Wert von pH 10 eingestellt.

Die Ausfärbungen erfolgten nach Abmischung im Gewichtsverhältnis 1:1 mit einer kommerziellen 6%igen Wasserstoffperoxid Entwickler-Zubereitung des Handelsprodukts Poly Color Brillance der Firma Schwarzkopf-Henkel auf Haarsträhnen des oben angegebenen Codes. Das Gewichtsverhältnis Farbstoffmischung : Haar betrug 4:1, die Einwirkzeit betrug 30 Minuten bei einer Temperatur von 32 Grad Celsius. Anschließend wurden die Strähnen gespült, getrocknet und mindestens 24 Stunden unter Raumbedingungen ruhen gelassen. Dann wurden die Strähnen farbmetrisch vermessen (*vide infra*).

1.2 **Farbabzug**

Als reduktive Entfärbemittel wurden folgende Zusammensetzungen gemäß Tabelle 1 durch Mischen hergestellt. Die Mengen sind, soweit nicht anders gekennzeichnet, Gew.-%.

Tabelle 1: reduktive Entfärbemittel

| Rohstoffe | V | E1* | E2* | E3 | E4 | E5 | E6 | E7 | E8* | E9* |
|---|---|---|---|---|---|---|---|---|---|---|
| $Na_2S_2O_4$ | 10,0 | 10,0 | 10,0 | 10,0 | 10,0 | 10,0 | 10,0 | 10,0 | 10,0 | 10,0 |
| Aerosil 200[4] | - | 5,0 | - | - | - | - | - | - | - | - |
| $\gamma$-Aluminiumoxid | - | - | 10,0 | - | - | - | - | - | - | 10,0 |
| Luviskol K 17 | - | - | - | 10,0 | 10,0 | - | - | - | - | - |
| Luviskol K 30 | - | - | - | - | - | 10,0 | - | - | - | - |
| Luviskol K 80 | - | - | - | - | - | - | 5,0 | - | - | - |
| Luviskol K 90 | - | - | - | - | - | - | - | 5,0 | - | - |
| Cucurbit[8]uril | - | - | - | - | - | - | - | - | 10,0 | - |
| Ascorbinsäure | - | - | - | - | 10,0 | - | - | - | - | 10,0 |
| Phosphorsäure | <------ ad pH 1,5 -----> | | | | | | | | | |
| Wasser | <----- ad 100 -----> | | | | | | | | | |
| Aerosil® 200 Kieselsäure (INCI-Bezeichnung: Silica) (Degussa)<br>* nicht erfindungsgemäss | | | | | | | | | | |

Die Untersuchungen zum Farbabzug wurden in Petrischalen von 9 cm Durchmesser bei Raumtemperatur durchgeführt. Dabei wurden pro zu testendem Entfärbemittel vier Haarsträhnen des gemäß Punkt 1.1 gefärbten Haars im Gewichtsverhältnis Entfärbemittel : Haar von 20:1 über eine Einwirkdauer von insgesamt 30 Minuten mit dem reduktiven Entfärbemittel in je einer Petrischale behandelt.

Nach Beendigung der Einwirkzeit wurde 1 Minute mit warmem fließendem Stadtwasser gespült, das Haar

anschließend 15 Minuten gefönt und unmittelbar danach farbmetrisch vermessen.

Weitere farbmetrische Messungen erfolgten nach 7 Tagen Lagerung des Haars bei Raumbedingungen.

**1.3 Farbmetrische Messungen**

Die Strähnen wurden farbmetrisch mit einem Farbmessgerät der Firma Datacolor, Typ Spectraflash 450 vermessen. Die Messgrößen sind die Lab-Werte. Hierbei steht der L-Wert für die Helligkeit (je geringer der L-Wert ist, desto größer ist die Farbintensität), während der a-Wert ein Maß für den Rotanteil einer Farbe ist (d.h. je größer der a-Wert ist, umso größer ist der Rotanteil). Der b-Wert ist ein Maß für den Blauanteil der Farbe, wobei der Blauanteil umso größer ist, je negativer der b-Wert ist. Je Strähne wurden vier Messpunkte vermessen. Abschließend wurden die arithmetischen Mittel der gemessenen Lab-Werte (L*, a*, b*) gebildet. Die Strähnen wurden wie beschrieben nach der Färbung, direkt nach der reduktiven Entfärbung und 7 Tage nach der reduktiven Entfärbung vermessen.

Als wesentliches Kriterium wurde der Farbabstand dE zwischen den gefärbten und reduktiv entfärbten Strähnen herangezogen. Dieser berechnet sich aus den jeweiligen L*a*b*-Mittelwerten mittels folgender Farbabstandsformel:

$$dE = \sqrt{(L^*_{entfärbt} - L^*_{gefärbt})^2 + (a^*_{entfärbt} - a^*_{gefärbt})^2 + (b^*_{entfärbt} - b^*_{gefärbt})^2}$$

Alle nachfolgend angegebenen Experimentalergebnisse belegen, dass durch Zusatz mindestens eines Adsorptionsmittels die Entfärbung verstärkt wird.

1.3.1 Entfärbung von Färbungen mit F1 (p-Toluylendiamin / 5-Amino-2-methylphenol)

| | V | E2 * | E3 | E4 | E8 * |
|---|---|---|---|---|---|
| dE nach 0 Tagen | 64,03 | 65,77 | 64,96 | 65,19 | 65,70 |
| dE nach 7 Tagen | 59,00 | 63,33 | 61,50 | 61,96 | 61,37 |

1.3.2 Entfärbung von Färbungen mit F2 (p-Toluylendiamin / 2,4-Diaminophenoxyethanol)

| | V | E1 * | E2 * | E5 | E6 | E7 | E8 * | E9 * |
|---|---|---|---|---|---|---|---|---|
| dE nach 0 Tagen | 50,41 | 54,38 | 51,01 | 52,21 | 51,41 | 52,72 | 50,61 | 52,36 |
| dE nach 7 Tagen | 45,87 | 50,87 | 48,76 | 49,01 | 49,57 | 48,60 | 49,62 | 48,97 |
| * nicht erfindungsgemäss | | | | | | | | |

1.3.3 Entfärbung von Färbungen mit F3 (p-Toluylendiamin / Resorcin)

| | V | E3 | E8 * | E9 * |
|---|---|---|---|---|
| dE nach 0 Tagen | 7,58 | 8,81 | 11,71 | 10,64 |
| dE nach 7 Tagen | 7,90 | 11,28 | 8,99 | 11,21 |

**Patentansprüche**

1. Mittel zum reduktiven Farbabzug, enthaltend in einem kosmetischen Träger mindestens ein Reduktionsmittel und mindestens ein Adsorptionsmittel, **dadurch gekennzeichnet, dass** das Adsorptionsmittel ausgewählt wird aus Homo- und/oder Copolymeren des Vinylpyrrolidons.

**2.** Mittel nach Anspruch 1, **dadurch gekennzeichnet, daß** das Reduktionsmittel ein Thionit-Radikal-Abspalter ist.

**3.** Mittel nach einem der Ansprüche 1 oder 2, **dadurch gekennzeichnet, daß** der Thionit-Radikal-Abspalter ausgewählt wird aus mindestens einem Vertreter aus der Gruppe, die gebildet wird aus Dithionit-Dianion $S_2O_4^{2-}$ und organischen Sulfinsäuren bzw. deren physiologisch verträglichen Salzen.

**4.** Mittel nach Anspruch 3, **dadurch gekennzeichnet, daß** als organische Sulfinsäure mindestens ein Sulfinsäure-Derivat der Formel (I) enthalten ist,

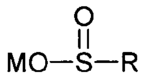 (I)

worin

M steht für ein Wasserstoffatom oder ein Äquivalent eines ein- oder mehrwertigen Kations,
R sich ableitet von einem Peptid oder für einen Rest gemäß einer der Formeln (II) bis (VI) steht,

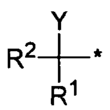 (II)

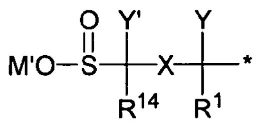 (III)

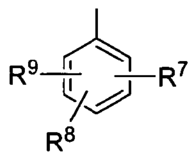 (IV)

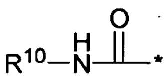 (V)

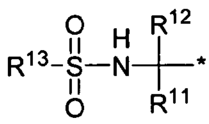 (VI)

worin bedeuten

Y und Y' unabhängig voneinander eine Hydroxygruppe, eine $-NH_2$ Gruppe oder eine Gruppe $-NR^3R$ , wobei $R^3$ und $R^4$ unabhängig voneinander für eine ($C_1$ bis $C_6$)-Alkylgruppe, eine ($C_2$ bis $C_6$)-Alkenylgruppe, eine ($C_1$ bis $C_6$)-Hydroxyalkylgruppe, eine ($C_2$ bis $C_6$)-Polyhydroxyalkylgruppe, eine Arylgruppe oder eine Aryl-($C_1$ bis $C_6$)-alkylgruppe stehen,
M' unabhängig von M die unter M aufgeführten Merkmale,
X eine direkte Bindung oder einen organischen Rest mit zwei freien Valenzen,
$R^1$ und $R^{14}$ unabhängig voneinander ein Wasserstoffatom, eine ($C_1$ bis $C_6$)-Alkylgruppe oder eine Carboxyalkylgruppe $-(CH_2)_m-COOM^{II}$, in der $M^{II}$ für ein Wasserstoffatom oder für ein Äquivalent eines ein- oder mehrwertigen Kations steht und m die Zahl 0, 1 oder 2 bedeutet,
$R^2$ ein Wasserstoffatom, eine ($C_1$ bis $C_6$)-Alkylgruppe, einen Carboxyalkylrest $-(CH_2)_n-COOM^{III}$ mit $M^{III}$ = Wasserstoffatom oder ein Äquivalent eines ein- oder mehrwertigen Kations und n eine ganze Zahl 0, 1, 2, 3, 4, 5 oder 6,
einen ($C_1$ bis $C_6$)-Alkyloxycarbonylrest, eine Sulfonsäuregruppe, eine Carbamoylgruppe, eine N,N-Di[($C_1$ bis $C_6$)-alkyl]carbamoylgruppe, eine N,N,N-Tri[($C_1$ bis $C_6$)-alkyl]ammonium-($C_1$ bis $C_6$)-alkylgruppe eine Carboxy-($C_2$ bis $C_6$)-alkenylgruppe, eine Cyano-($C_1$ bis $C_6$)-alkylgruppe oder eine ($C_1$ bis $C_6$)-Alkoxycarbonyl-($C_1$ bis $C_6$)-alkylgruppe, einen aliphatischen oder aromatischen Heterozyklus, der substituiert sein kann, oder
einen Rest gemäß Formel (IV) oder
$R^2$ zusammen mit $R^1$ und dem Restmolekül einen aliphatischen 5-, 6-, oder 7-gliedrigen Ring bildet, welcher mindestens ein kationisches, quaterniertes Stickstoffatom als Heteroatom enthält, wobei die kationische Ladung gegebenenfalls durch ein Äquivalent eines ein- oder mehrwertigen Anions kompensiert wird,
$R^7$ eine Carboxygruppe, eine Sulfonsäuregruppe, eine ($C_1$ bis $C_6$)-Alkoxycarbonylgruppe, eine Sulfonamidgruppe, eine Cyanogruppe, eine Nitrogruppe, eine Carboxy-($C_1$ bis $C_6$)-alkylgruppe, eine Carboxy-($C_1$ bis $C_6$)-alkoxygruppe oder eine Gruppe $-N^+R^IR^{II}R^{III}$,

mit $R^I$, $R^{II}$ und $R^{III}$ stehen unabhängig voneinander für eine ($C_1$ bis $C_6$)-Alkylgruppe, eine ($C_2$ bis $C_6$)-Alkenylgruppe, eine Aryl($C_1$ bis $C_6$)-alkylgruppe oder eine ($C_1$ bis $C_6$)-Hydroxyalkylgruppe,
$R^8$ und $R^9$ unabhängig voneinander ein Wasserstoffatom, eine ($C_1$ bis $C_6$)-Alkylgruppe, eine ($C_2$ bis $C_6$)-Alkenylgruppe, eine ($C_1$ bis $C_6$)-Hydroxyalkylgruppe, eine ($C_2$ bis $C_6$)-Polyhydroxyalkylgruppe, eine ($C_1$ bis $C_6$)-Alkoxygruppe, eine Hydroxygruppe, eine Aminogruppe, eine Carboxygruppe, eine Nitrogruppe, eine Cyanogruppe oder ein Halogenatom,
$R^{10}$ eine ($C_1$ bis $C_6$)-Alkylgruppe, eine gegebenenfalls substituierte Arylgruppe, eine gegebenenfalls substituierte Heteroarylgruppe, eine Carboxy-($C_1$ bis $C_6$)-alkylgruppe, eine Carboxy-($C_2$ bis $C_6$)-alkenylgruppe, eine ($C_1$ bis $C_6$)-Alkoxycarbonylgruppe oder eine ($C_1$ bis $C_6$)-Alkoxycarbonyl-($C_1$ bis $C_6$)-alkylgruppe,
$R^{11}$, $R^{12}$ und $R^{13}$ unabhängig voneinander ein Wasserstoffatom, eine ($C_1$ bis $C_6$)-Alkylgruppe, eine ($C_2$ bis $C_6$)-Alkenylgruppe, eine Perfluor-($C_1$ bis $C_6$)-alkylgruppe, eine ($C_3$ bis $C_6$)-Cycloalkylgruppe, eine gegebenenfalls substituierte Arylgruppe, eine gegebenenfalls substituierte Heteroarylgruppe, eine ($C_1$ bis $C_6$)-Hydroxyalkylgruppe, eine ($C_2$ bis $C_6$)-Polyhydroxyalkylgruppe, eine Aryl-($C_1$ bis $C_6$)-alkylgruppe, eine Carboxy-($C_1$ bis $C_6$)-alkylgruppe, eine Carboxy-($C_2$ bis $C_6$)-alkenylgruppe oder eine ($C_1$ bis $C_6$)-Alkoxycarbonyl-($C_1$ bis $C_6$)-alkylgruppe,

mit der Maßgabe, dass in Formel (II) $R^1$ und $R^2$ nicht gleichzeitig für ein Wasserstoffatom stehen.

in Kombination mit mindestens einer Verbindung, besitzend mindestens ein quartäres Stickstoffatom.

**5.** Mittel nach Anspruch 4, **dadurch gekennzeichnet, daß** gemäß Formel (I) die Reste Y der Formel (II) bzw. der Formel (III) und Y' der Formel (III) unabhängig voneinander eine Hydroxygruppe oder eine Gruppe $-NH_2$ bedeuten.

**6.** Mittel nach einem der Ansprüche 4 oder 5, **dadurch gekennzeichnet, daß** der Rest R für eine Gruppe der Formel (II) steht.

**7.** Mittel nach einem der Ansprüche 1 bis 6, **dadurch gekennzeichnet, daß** die Reduktionsmittel in einder Menge von 2 bis 20 Gew.-%, besonders bevorzugt von 5 bis 10 Gew.%, jeweils bezogen auf das Gewicht des Mittels, enthalten sind.

**8.** Mittel nach einem der Ansprüche 1 bis 7, **dadurch gekennzeichnet, daß** die Adsorptionsmittel in einder Menge von 2 bis 20 Gew.-%, besonders bevorzugt von 5 bis 10 Gew.-%, jeweils bezogen auf das Gewicht des Mittels, enthalten sind.

**9.** Mittel nach einem der Ansprüche 1 bis 8, **dadurch gekennzeichnet, daß** die Adsorptionsmittel und die Reduktionsmittel in einem Gewichtsverhältnis (Adsorptionsmittel zu Reduktionsmittel) von 1 zu 2 bis 2 zu 1, insbesondere von 1.2 zu 1 bis 1 zu 1.2 enthalten sind.

**10.** Verwendung eines Mittels gemäß einem der Ansprüche 1 bis 9 zur Entfärbung von Haut und/oder Haaren, die mit Oxidationsfarbstoffen und/oder direktziehenden Farbstoffen gefärbt sind.

**11.** Verfahren zur reduktiven Entfärbung von jeweils mit Oxidationsfarbstoffen und/oder direktziehenden Farbstoffen eingefärbter Haut und/oder eingefärbten Haaren, **dadurch gekennzeichnet, daß** ein Mittel gemäß einem der Ansprüche 1 bis 9 auf das entsprechende eingefärbte Substrat aufgetragen wird und nach einer Einwirkzeit wieder abgespült wird.

## Claims

**1.** An agent for reductive colour removal, containing in a cosmetic carrier at least one reducing agent and at least one adsorbent, **characterised in that** the adsorbent is selected from homo- and/or copolymers of vinylpyrrolidone.

**2.** An agent according to claim 1, **characterised in that** the reducing agent is a thionite free-radical releaser.

**3.** An agent according to either of claim 1 or claim 2, **characterised in that** the thionite free-radical releaser is selected from at least one representative of the group formed of dithionite dianion $S_2O_4^{2-}$ and organic sulfinic acids or the physiologically acceptable salts thereof.

**4.** An agent according to claim 3, **characterised in that** at least one sulfinic acid derivative of formula (I) is present as the organic sulfinic acid,

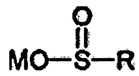
(I)

in which

M denotes a hydrogen atom or an equivalent of a mono- or polyvalent cation,
R is derived from a peptide or denotes a residue according to one of formulae (II) to (VI),

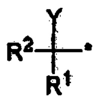
(II)
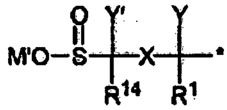
(III)
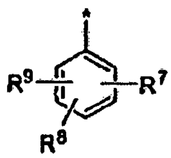
(IV)
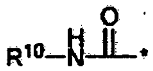
(V)
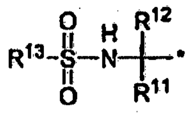
(VI)

in which

Y and Y' mutually independently mean a hydroxyl group, an $-NH_2$ group or an $-NR^3R^4$ group, wherein $R^3$ and $R^4$ mutually independently denote a ($C_1$ to $C_6$) alkyl group, a ($C_2$ to $C_6$) alkenyl group, a ($C_1$ to $C_6$) hydroxyalkyl group, a ($C_2$ to $C_6$) polyhydroxyalkyl group, an aryl group, or an aryl-($C_1$ to $C_6$)-alkyl group,
M' means, independently of M, the features listed under M,
X means a direct bond or an organic residue with two free valences,
$R^1$ and $R^{14}$ mutually independently mean a hydrogen atom, a ($C_1$ to $C_6$) alkyl group, or a carboxyalkyl group $-(CH_2)_m-COOM^{II}$, in which $M^{II}$ denotes a hydrogen atom or an equivalent of a mono- or polyvalent cation and m means the number 0, 1 or 2,
$R^2$ means a hydrogen atom, a ($C_1$ to $C_6$) alkyl group, a carboxyalkyl residue $-(CH_2)_n-COOM^{III}$ where $M^{III}$ = hydrogen atom or an equivalent of a mono- or polyvalent cation and n an integer 0, 1, 2, 3, 4, 5 or 6, a ($C_1$ to $C_6$) alkyloxycarbonyl residue, a sulfonic acid group, a carbamoyl group, an N,N-di-[($C_1$ to $C_6$)-alkyl] carbamoyl group, an N,N,N-tri-[($C_1$ to $C_6$)-alkyl]ammonium-($C_1$ to $C_6$)-alkyl group, a carboxy-($C_2$ to $C_6$)-alkenyl group, a cyano-($C_1$ to $C_6$)-alkyl group or a ($C_1$ to $C_6$)-alkoxycarbonyl-($C_1$ to $C_6$)-alkyl group, an aliphatic or aromatic heterocycle, which may be substituted,
or a residue according to formula (IV) or
$R^2$ together with $R^1$ and the remainder of the molecule form an aliphatic 5-, 6-, or 7-membered ring which contains at least one cationic, quaternised nitrogen atom as heteroatom, wherein the cationic charge is optionally compensated by an equivalent of a mono- or polyvalent anion,
$R^7$ means a carboxy group, a sulfonic acid group, a ($C_1$ to $C_6$) alkoxycarbonyl group, a sulfonamide group, a cyano group, a nitro group, a carboxy-($C_1$ to $C_6$)-alkyl group, a carboxy-($C_1$ to $C_6$)-alkoxy group or an $-N+R^IR^{II}R^{III}$ group, where $R^I$, $R^{II}$ and $R^{III}$ mutually independently denote a ($C_1$ to $C_6$) alkyl group, a ($C_2$ to $C_6$) alkenyl group, an aryl-($C_1$ to $C_6$)-alkyl group, or a ($C_1$ to $C_6$) hydroxyalkyl group,
$R^8$ and $R^9$ mutually independently mean a hydrogen atom, a ($C_1$ to $C_6$) alkyl group, a ($C_2$ to $C_6$) alkenyl group, a ($C_1$ to $C_6$) hydroxyalkyl group, a ($C_2$ to $C_6$) polyhydroxyalkyl group, a ($C_1$ to $C_6$) alkoxy group, a hydroxyl group, an amino group, a carboxy group, a nitro group, a cyano group or a halogen atom,
$R^{10}$ means a ($C_1$ to $C_6$) alkyl group, an optionally substituted aryl group, an optionally substituted heteroaryl group, a carboxy-($C_1$ to $C_6$)-alkyl group, a carboxy-($C_2$ to $C_6$)-alkenyl group, a ($C_1$ to $C_6$)-alkoxycarbonyl group or a ($C_1$ to $C_6$)-alkoxycarbonyl-($C_1$ to $C_6$)-alkyl group,
$R^{11}$, $R^{12}$ and $R^{13}$ mutually independently mean a hydrogen atom, a ($C_1$ to $C_6$) alkyl group, a ($C_2$ to $C_6$) alkenyl group, a perfluoro-($C_1$ to $C_6$)-alkyl group, a ($C_3$ to $C_6$)-cycloalkyl group, an optionally substituted

aryl group, an optionally substituted heteroaryl group, a ($C_1$ to $C_6$) hydroxyalkyl group, a ($C_2$ to $C_6$) polyhydroxyalkyl group, an aryl-($C_1$ to $C_6$)-alkyl group, a carboxy-($C_1$ to $C_6$)-alkyl group, a carboxy-($C_2$ to $C_6$)-alkenyl group or a ($C_1$ to $C_6$)-alkoxycarbonyl-($C_1$ to $C_6$)-alkyl group,

with the proviso that in formula (II) $R^1$ and $R^2$ do not simultaneously denote a hydrogen atom,

in combination with at least one compound having at least one quaternary nitrogen atom.

5. An agent according to claim 4, **characterised in that** according to formula (I) the residues Y of formula (II) or of formula (III) and Y' of formula (III) mutually independently mean a hydroxyl group or an $-NH_2$ group.

6. An agent according to either of claim 4 or claim 5, **characterised in that** the residue R denotes a group of the formula (II).

7. An agent according to any one of claims 1 to 6, **characterised in that** the reducing agents are present in a quantity of 2 to 20 wt.%, particularly preferably of 5 to 10 wt.%, in each case relative to the weight of the agent.

8. An agent according to any one of claims 1 to 7, **characterised in that** the adsorbents are present in a quantity of 2 to 20 wt.%, particularly preferably of 5 to 10 wt.%, in each case relative to the weight of the agent.

9. An agent according to any one of claims 1 to 8, **characterised in that** the adsorbents and the reducing agents are present in a weight ratio (of adsorbent to reducing agent) of 1:2 to 2:1, in particular of 1.2:1 to 1:1.2.

10. Use of an agent according to any one of claims 1 to 9 for decolourisation of skin and/or hair which are dyed with oxidation dyes and/or substantive dyes.

11. A method for reductive decolourisation of skin and/or hair in each case dyed with oxidation dyes and/or substantive dyes, **characterised in that** an agent according to any one of claims 1 to 9 is applied to the corresponding dyed substrate and rinsed off again after a period of exposure.

## Revendications

1. Agent pour la décoloration par réduction, contenant, dans un support cosmétique, au moins un agent de réduction et au moins un agent d'adsorption, **caractérisé en ce que** l'agent d'adsorption est choisi parmi des homopolymères et/ou des copolymères de la vinylpyrrolidone.

2. Agent selon la revendication 1, **caractérisé en ce que** l'agent de réduction est un agent de libération de radicaux thionites.

3. Agent selon l'une quelconque des revendications 1 ou 2, **caractérisé en ce que** l'agent de libération de radicaux thionites est choisi parmi au moins un représentant un groupe qui est formé par un dianion de dithionite $S_2O_4^{2-}$ et des acides sulfiniques organiques, respectivement leurs sels physiologiquement acceptables.

4. Agent selon la revendication 3, **caractérisé en ce qu'**il contient, à titre d'acide sulfinique organique au moins un dérivé de l'acide sulfinique répondant à la formule (I)

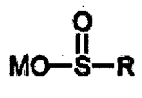
(I)

dans laquelle

M représente un atome d'hydrogène ou un équivalent d'un cation monovalent ou polyvalent ;
R dérive d'un peptide ou d'un résidu répondant à une des formules (II) à (VI)

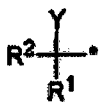 (II) 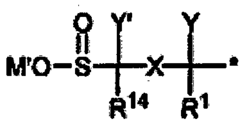 (III) 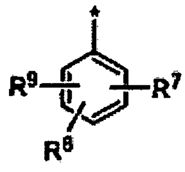 (IV)

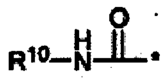 (V) 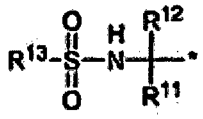 (VI)

dans lesquelles

- Y et Y' représentent, indépendamment l'un de l'autre, un groupe hydroxyle, un groupe $-NH_2$ ou un groupe $-NR^3R^4$ dans lequel $R^3$ et $R^4$ représentent, indépendamment l'un de l'autre, un groupe alkyle en $C_1$-$C_6$, un groupe alcényle en $C_2$-$C_6$, un groupe hydroxyalkyle en $C_1$-$C_6$, un groupe polyhydroxyalkyle en $C_2$-$C_6$, un groupe aryle ou un groupe aryl(alkyle en $C_1$-$C_e$) ;
- M' représente, indépendamment de M, les caractéristiques indiquées sous M ;
- X représente une liaison directe ou un résidu organique comportant deux valences libres ;
- $R^1$ et $R^{14}$ représentent, indépendamment l'un de l'autre, un atome d'hydrogène, un groupe alkyle en $C_1$-$C_6$ ou un groupe carboxyalkyle $-(CH_2)_m-COOM^{II}$ dans lequel $M^{II}$ représente un atome d'hydrogène ou un équivalent d'un cation monovalent ou polyvalent, et m représente le nombre 0, 1 ou 2 ;
- $R^2$ représente un atome d'hydrogène, un groupe alkyle en $C_1$-$C_6$, un résidu carboxyalkyle $-(CH_2)_n-COOM^{III}$ dans lequel
- $M^{III}$ représente un atome d'hydrogène ou un équivalent d'un cation monovalent ou polyvalent, et n représente un nombre entier égal à 0, 1, 2, 3, 4, 5 ou 6;
- un résidu (alkyle en $C_1$-$C_6$)oxycarbonyle, un groupe d'acide sulfonique, un groupe carbamoyle, un groupe N,N-di(alkyle en $C_1$-$C_6$)carbamoyle, un groupe N,N,N-tri(alkyle en $C_1$-$C_6$)ammonium(alkyle en $C_1$-$C_6$), un groupe carboxy(alcényle en $C_2$-$C_6$), un groupe cyano(alkyle en $C_1$-$C_6$) ou un groupe (alcoxy en $C_1$-$C_6$) carbonyl(alkyle en $C_1$-$C_6$), un hétérocycle aliphatique ou aromatique qui peut être substitué ; ou
- un résidu répondant à la formule (IV) ; ou bien
- $R^2$ ensemble avec $R^1$ et la molécule résiduelle forment un noyau aliphatique de 5, 6 ou 7 membres, qui contient au moins, à titre d'hétéroatome, un atome d'azote cationique quaternisé, la charge cationique étant compensée de manière facultative par un équivalent d'un anion monovalent ou polyvalent ;
- $R^7$ représente un groupe carboxyle, un groupe d'acide sulfonique, un groupe (alcoxy en $C_1$-$C_6$)carbonyle, un groupe sulfonamide, un groupe cyano, un groupe nitro, un groupe carboxy(alkyle en $C_1$-$C_6$), un groupe carboxy(alcoxy en $C_1$-$C_6$) ou un groupe $-N^+R^IR^{II}R^{III}$ ;
- dans lequel $R^I$, $R^{II}$ et $R^{III}$ représentent, indépendamment l'un de l'autre, un groupe alkyle en $C_1$-$C_6$, un groupe alcényle en $C_2$-$C_6$, un groupe aryl(alkyle en $C_1$-$C_6$) ou un groupe hydroxyalkyle en $C_1$-$C_6$ ;
- $R^8$ et $R^9$ représentent, indépendamment l'un de l'autre, un atome d'hydrogène, un groupe alkyle en $C_1$-$C_6$, un groupe alcényle en $C_2$-$C_6$, un groupe hydroxyalkyle en $C_1$-$C_6$, un groupe polyhydroxyalkyle en $C_2$-$C_6$, un groupe alcoxy en $C_1$-$C_6$, un groupe hydroxyle, un groupe amino, un groupe carboxyle, un groupe nitro, un groupe cyano ou un atome d'halogène ;
- $R^{10}$ représente un groupe alkyle en $C_1$-$C_6$, un groupe aryle substitué de manière facultative, un groupe hétéroaryle substitué de manière facultative, un groupe carboxy(alkyle en $C_1$-$C_6$), un groupe carboxy(alcényle en $C_2$-$C_6$), un groupe (alcoxy en $C_1$-$C_6$)carbonyle ou un groupe (alcoxy en $C_1$-$C_6$)carbonyl(alkyle en $C_1$-$C_6$) ;
- $R^{11}$, $R^{12}$ et $R^{13}$ représentent, indépendamment l'un de l'autre, un atome d'hydrogène, un groupe alkyle en $C_1$-$C_6$, un groupe alcényle en $C_2$-$C_6$, un groupe perfluoroalkyle en $C_1$-$C_6$, un groupe cycloalkyle en $C_3$-$C_6$, un groupe aryle substitué de manière facultative, un groupe hétéroaryle substitué de manière facultative, un groupe hydroxyalkyle en $C_1$-$C_6$, un groupe polyhydroxyalkyle en $C_2$-$C_6$, un groupe aryl(alkyle en $C_1$-$C_6$), un groupe carboxy(alkyle en $C_1$-$C_6$), un groupe carboxy(alcényle en $C_2$-$C_6$) ou un groupe (alcoxy en $C_1$-$C_6$) carbonyl(alkyle en $C_1$-$C_6$) ;

avec cette mesure que, dans la formule (II), $R^1$ et $R^2$ ne peuvent pas représenter simultanément un atome d'hydrogène ;

en combinaison avec au moins un composé possédant au moins un atome d'azote quaternaire.

5. Agent selon la revendication 4, **caractérisé en ce que**, conformément à la formule (I), les résidus Y de la formule (II) respectivement de la formule (III) et Y' de la formule (III) représentent, indépendamment l'un de l'autre, un groupe hydroxyle ou un groupe $-NH_2$.

6. Agent selon la revendication 4 ou 5, **caractérisé en ce que** le résidu R représente un groupe répondant à la formule (II).

7. Agent selon l'une quelconque des revendications 1 à 6, **caractérisé en ce qu'**il contient les agents de réduction en une quantité de 2 à 20 % en poids, de manière particulièrement préférée de 5 à 10 % en poids, chaque fois rapportés au poids de l'agent.

8. Agent selon l'une quelconque des revendications 1 à 7, **caractérisé en ce qu'**il contient les agents d'adsorption en une quantité de 2 à 20 % en poids, de manière particulièrement préférée de 5 à 10 % en poids, chaque fois rapportés au poids de l'agent.

9. Agent selon l'une quelconque des revendications 1 à 8, **caractérisé en ce qu'**il contient les agents d'adsorption et les agents de réduction dans un rapport pondéral (de l'agent d'adsorption à l'agent de réduction) de 1 - 2 à 2 - 1, en particulier de 1,2 - 1 à 1 - 1,2.

10. Utilisation d'un agent selon l'une quelconque des revendications 1 à 9, pour la décoloration de la peau et/ou des cheveux, qui ont été colorés avec des colorants d'oxydation et/ou avec des colorants directs.

11. Procédé pour la décoloration par réduction de la peau et/ou des cheveux respectivement colorés avec des colorants d'oxydation et/ou des colorants directs, **caractérisé en ce qu'**on applique un agent selon l'une quelconque des revendications 1 à 9, sur le substrat coloré de manière correspondante, avant de l'en éliminer à nouveau par rinçage après l'avoir laissé agir pendant un certain temps.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3892845 A
- DE OS1617829 A
- EP 1079018 A
- WO 9918067 A1
- WO 0230369 A1
- WO 03026597 A1
- WO 0341668 A1
- DE PS4413686 C
- DE 19717281 A1
- GB 1026978 A
- GB 1153196 A
- DE 2359399
- JP 02019576 A
- WO 9615765 A
- DE 3843892
- DE 4133957
- WO 9408969 A
- WO 9408970 A
- EP 740931 A
- DE 19543988
- EP 998908 A2
- DE 3725030 A
- DE 3723354 A
- DE 3926344 A